(19) Europäisches Patentamt
European Patent Office
Office européen des brevets

(11) **EP 3 997 416 B1**

(12) **FASCICULE DE BREVET EUROPEEN**

(45) Date de publication et mention de la délivrance du brevet:
**15.05.2024 Bulletin 2024/20**

(21) Numéro de dépôt: **20753397.7**

(22) Date de dépôt: **10.07.2020**

(51) Classification Internationale des Brevets (IPC):
*G01B 21/08* (2006.01)   *B29C 49/78* (2006.01)
*G01B 11/06* (2006.01)   *G01N 21/90* (2006.01)
*G01N 25/72* (2006.01)   *G01N 33/38* (2006.01)
*G01J 5/00* (2022.01)

(52) Classification Coopérative des Brevets (CPC):
**G01B 21/085; G01B 11/06; G01N 21/90;
G01N 33/386;** G01N 25/72

(86) Numéro de dépôt international:
**PCT/FR2020/051257**

(87) Numéro de publication internationale:
**WO 2021/009456 (21.01.2021 Gazette 2021/03)**

(54) **INSTALLATION ET PROCEDE POUR MESURER L'EPAISSEUR DES PAROIS DE RECIPIENTS EN VERRE**

VORRICHTUNG UND VERFAHREN ZUM MESSEN DER DICKE DER WÄNDE VON GLASBEHÄLTERN

EQUIPMENT AND METHOD FOR MEASURING THE THICKNESS OF THE WALLS OF GLASS CONTAINERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.07.2019 FR 1907877**

(43) Date de publication de la demande:
**18.05.2022 Bulletin 2022/20**

(73) Titulaire: **TIAMA
69230 Saint-Genis-Laval (FR)**

(72) Inventeurs:
• **LECONTE, Marc
69700 LOIRE SUR RHONE (FR)**
• **SOLANE, Pierre-Yves
69004 LYON (FR)**

(74) Mandataire: **Cabinet Beau de Loménie
51 avenue Jean Jaurès
BP 7073
69301 Lyon Cedex 07 (FR)**

(56) Documents cités:
**FR-A1- 2 842 301     US-A1- 2019 195 619
US-B1- 6 188 079**

• **Crystran: "Silica Glass (SiO2) Optical Material", , 12 décembre 2016 (2016-12-12), XP055735537, Extrait de l'Internet: URL:https://web.archive.org/web/20161212204800/https://www.crystran.co.uk/optical-materials/silica-glass-sio2 [extrait le 2020-09-30]**

EP 3 997 416 B1

**Description**

Domaine Technique

La présente invention concerne le domaine technique de l'inspection optique de récipients ou d'objets creux translucides ou transparents présentant une haute température.

L'objet de l'invention vise plus précisément l'inspection optique à haute cadence d'objets tels que des bouteilles ou des flacons en verre encore chauds sortant d'une machine de fabrication ou de formage. Ainsi, l'objet de l'invention vise l'inspection d'objets dans le secteur chaud d'une installation de fabrication.

De manière classique, après fusion du verre dans un four autour de 1600°, le verre fondu est amené au-dessus des machines de formage par l'intermédiaire de canaux dits « avant-coeur ». L'installation comporte également un distributeur de paraisons de verre fondu ou gouttes de verre malléable tombant par gravité dans chaque moule ébaucheur. Ainsi cet organe forme des gouttes de verres qui sont distribuées en direction de diverses sections indépendantes de formage par l'intermédiaire d'un ensemble de guides appelés délivre. Une machine de formage dite machine IS est constituée de différentes sections équipées chacune d'au moins une cavité d'ébauchage équipée d'un moule ébaucheur, et du même nombre de cavités de finition, recevant chacune un moule finisseur dans lequel les récipients prennent leur forme finale à haute température. Les 1, 2 3 ou 4 cavités d'ébauche d'une section sont chargées, à tour de rôle selon un ordre prédéfini, en une, deux, trois ou quatre gouttes de verre chaud (environ 1200°C) appelées « gob ». En sortie de la machine de formage, les récipients encore à haute température, généralement entre 300°C et 600°C, sont pris en charge au niveau de leur bague pour être acheminés de manière à constituer une file sur un convoyeur de transport. L'espacement entre les récipients est variable et imposé par la machine de formage selon son propre entraxe et les diamètres des récipients. Le convoyeur de transport amène les récipients à défiler successivement dans divers postes de traitement tels qu'une hotte de pulvérisation d'un traitement de surface et un four de recuit appelée arche de recuisson.

Il apparaît intéressant d'identifier un défaut de formage le plus tôt possible à la sortie de la machine de formage avant les divers postes de traitement de manière à pouvoir le corriger le plus tôt possible au niveau de la machine de formage. Il est ainsi avantageux de détecter notamment des écarts dimensionnels ou des déformations des récipients qui sont directement reliés à des réglages du procédé de formage, afin de corriger, en cas de dérive, le procédé le plus rapidement possible.

Le contrôle de la qualité de tels récipients permet d'éliminer ceux qui présentent des défauts susceptibles d'affecter leur caractère esthétique ou plus grave, de constituer un réel danger pour les utilisateurs ultérieurs. Ainsi, il apparaît nécessaire de contrôler la qualité de la répartition d'épaisseur de tels récipients de manière à éliminer les récipients présentant des épaisseurs trop faibles ou des différences d'épaisseurs dans certaines zones susceptibles d'affecter la résistance mécanique.

En effet, la qualité de la répartition d'épaisseur est un paramètre très important, car l'épaisseur peut être variable, trop forte ou trop faible en différentes parties du récipient. Cette disparité d'épaisseur pose un problème car les articles produits sont potentiellement fragiles. De plus, il est recherché par les fabricants la possibilité de fabriquer des récipients en verre allégés et amincis, il faut donc savoir correctement répartir le verre. Les paramètres de fabrication qui influencent la répartition de verre sont connus et nombreux, il est nécessaire de les maîtriser. Il s'agit notamment, cités à titre d'exemples connus, les paramètres suivants :

i) les paramètres de chargement, qui comprennent le vecteur vitesse et le centrage de la goutte lorsqu'elle tombe dans le moule ébaucheur ;
ii) la répartition de la chaleur dans la goutte ;
iii) le refroidissement des moules.

Pour agir correctement sur les paramètres de fabrication, il est nécessaire de connaître la répartition de verre des récipients immédiatement après le formage.

Il doit être compris que connaître la répartition d'épaisseur signifie connaître de manière absolue les épaisseurs en différents points du récipient ou à défaut de manière relative des écarts d'épaisseur entre différentes régions du récipient. Une bouteille est par exemple composée de bas en haut, d'un fond, d'un corps relié au fond par le talon, puis d'un col, relié au corps par une épaule et enfin une bague destinée au remplissage et à la fermeture par un bouchon, une capsule ou un couvercle. Un défaut de répartition de verre peut être observé par exemple verticalement, avec un excès de verre au fond, et un mince à l'épaule. On peut également observer des défauts de répartition horizontale, avec par exemple au niveau de l'épaule, plus de verre d'un côté qu'à l'opposé par rapport à l'axe. Cette analyse non seulement de valeurs minimales et maximales de l'épaisseur de verre, mais également la répartition et des écarts verticaux ou horizontaux, ainsi que l'emplacement des zones minces ou épaisses, est significative pour corriger correctement le procédé.

# EP 3 997 416 B1

Technique antérieure

Dans l'état de la technique, diverses solutions utilisant le rayonnement infrarouge émis par les récipients encore chauds ont été proposées pour leur inspection en sortie de la machine de formage en vue de mesurer la répartition du verre.

Par exemple, le brevet US 3 535 522 décrit un procédé pour mesurer l'épaisseur de verre d'un récipient consistant à mesurer le rayonnement infrarouge émis par un tel récipient en sortie de la machine de formage. La mesure du rayonnement infrarouge est réalisée alors que le récipient est placé dans un four pour homogénéiser la température du récipient à une valeur déterminée. Ensuite, le récipient toujours à l'intérieur du four, est mis en rotation autour d'un axe vertical, devant l'axe optique d'un capteur infrarouge qui mesure durant un tour, entre 2.06 à 2.5 microns ou bien entre 3.56 à 4.06 microns, ce rayonnement traversant la paroi. La température étant supposées rendue homogène grâce au four, les variations de rayonnement perçues sont imputables directement aux variations d'épaisseur. Cette technique ne permet pas de contrôler en continu les récipients et nécessite une manipulation des récipients conduisant à un procédé lent et susceptible d'entraîner des déformations des récipients.

Le brevet EP 0 643 297 décrit un dispositif permettant d'effectuer une analyse et un diagnostic sur un procédé de fabrication de produits en verre comportant un capteur sensible au rayonnement infrarouge émis par les objets sortant de la machine de formage. Ce système comporte également un dispositif de traitement numérique comparant le rayonnement à un modèle de référence mathématique afin de déterminer les écarts existants dans la distribution du verre et/ou les causes conduisant à la présence de contraintes thermiques dans le récipient. Par ailleurs ce brevet ne fournit aucune indication sur les moyens d'obtenir un modèle mathématique de référence.

Il faut prendre en compte qu'un tel dispositif de mesure infrarouge est donc installé pour observer les récipients qui défilent sur le convoyeur de sortie en aval (dans le sens de défilement) de la machine de fabrication, c'est -à-dire en aval de la dernière section, qui se trouve la plus proche du dispositif. A l'autre extrémité de la machine se trouve donc la section la plus en amont. Concernant le rayonnement émis par les récipients, il dépend de nombreux paramètres, dont la répartition de matière mais aussi la répartition de température. Durant le transfert des récipients entre la section et le dispositif de mesure infrarouge, des transferts de température ont lieu par rayonnement et conduction entre les différentes portions du récipient, allant dans le sens de l'équilibre thermique, pouvant être appelés « l'homogénéisation spontanée » de température, et un refroidissement global par rayonnement et convection, appelé « le refroidissement ». L'état thermique des récipients, donc la répartition de la température dans le matériau du récipient lors de leur sortie des moules, est appelé dans la suite « conditions initiales ». L'état thermique des récipients au moment de leur inspection dépend donc d'une part desdites conditions initiales, et d'autre part de l'homogénéisation spontanée et du refroidissement durant le transport, lesquels sont évidemment différents en fonction de la distance parcourue par le récipient depuis sa section de formage jusqu'au poste d'inspection.

En pratique, selon ce brevet antérieur on détecte des variations de rayonnement dues à des écarts de contraintes thermiques ou d'épaisseur, mais il est impossible de déterminer une valeur de contrainte ou de l'épaisseur, et même de déterminer si une variation de rayonnement est liée à un écart de contraintes thermiques ou d'épaisseur de matériau. Il apparaît donc en pratique impossible, de mettre en oeuvre une telle technique dans la mesure où la mesure du rayonnement infrarouge dépend de nombreux paramètres tels que ceux énumérés à titre d'exemple non limitatif ci-après : L'intensité du rayonnement infrarouge émis par les récipients chauds dépend fortement de la température suivant la loi de Stefan BOLTZMAN : $E = sT^4$ où E = Quantité totale de radiation émise par un objet en (watts m-2), s = la constante de Stefan BOLTZMAN = 5.67 xl0-8 Watts m-2K-4 et T = la température en degrés Kelvin (K).

L'intensité du rayonnement infrarouge émis par les récipients chauds dépend des caractéristiques de ces récipients chauds tels que par exemple la taille, la couleur, la forme et la composition du verre.

Il doit être considéré que la distance entre le capteur infrarouge et la sortie des moules est différente d'un moule à l'autre de sorte que le temps de refroidissement pour chaque récipient chaud est différent, de sorte que les récipients chauds présentent des températures différentes lorsqu'ils passent devant le capteur infrarouge. En d'autres termes, l'intensité du rayonnement infrarouge mesurée par le capteur dépend de l'origine du moule de fabrication et plus précisément de la position de ce moule par rapport au capteur.

En sortie de la machine de formage, les récipients sont placés par glissement sur un convoyeur. Il s'ensuit une différence de positionnement des récipients sur le convoyeur par rapport au capteur de mesure infrarouge, ce qui est à même de modifier les mesures prises.

Les conditions de température de formage ainsi que les interactions pendant le convoyage des récipients peuvent varier en fonction des conditions de production (mise en route, incident ...) et de l'environnement (jour/nuit, météo, courant d'air ...).

Puisque le rayonnement utilisé selon EP 0 643 297 est traversant, le rayonnement perçu est celui des deux parois combinées.

Il résulte de ce qui précède que de nombreux paramètres influencent le rayonnement infrarouge de sorte qu'un tel brevet n'apporte pas une solution pour mesurer la répartition de l'épaisseur de verre pour des récipients à haute

3

température. Ce brevet enseigne simplement de détecter des écarts dans la répartition du verre, à condition qu'un opérateur vérifie que les écarts de rayonnement sont dus à l'épaisseur. Seulement des valeurs relatives d'épaisseurs ou de contraintes thermiques sont estimées, entre différentes régions des récipients ou entre différents récipients et sur de courtes périodes temporelles. Ce brevet ne permet pas de mesurer en valeur absolue l'épaisseur de verre des récipients quel que soit le moment de la prise de mesures.

Selon une variante de réalisation, ce brevet prévoit la mise en oeuvre d'un capteur optique permettant de faire des images des produits en verre afin d'obtenir des informations sur des déviations et/ou la distribution du verre. Les informations sont comparées avec les données obtenues à partir du capteur sensible au rayonnement infrarouge de manière à pouvoir ajuster les critères selon lesquels les données fournies par le capteur sensible au rayonnement infrarouge ont été analysées. Si la mise en oeuvre de cette variante de réalisation apporte une correction aux critères utilisés, elle ne permet pas de remédier aux inconvénients inhérents à la méthode décrite dans ce brevet et rappelés ci-dessus. Aussi, cette solution ne permet pas de mesurer l'épaisseur du verre, ni en valeur relative ni en valeur absolue et par suite la répartition de l'épaisseur sur une zone étendue et encore moins sur tout le récipient.

Le brevet EP 1 020 703 propose de mesurer l'épaisseur de verre d'un récipient à partir du rayonnement infrarouge consistant à mesurer une première intensité dudit rayonnement dans une première bande spectrale à laquelle le rayonnement est émis par la matière entre les deux surfaces externe et interne du récipient. La première bande spectrale dont le signal dépend à la fois de la température de verre et de l'épaisseur est de préférence entre 0.4 et 1.1 micron. Le procédé consiste également à mesurer une deuxième intensité dudit rayonnement dans une deuxième bande spectrale à laquelle le rayonnement est émis substantiellement entièrement par une seule surface externe du récipient. Selon ce brevet, la deuxième bande spectrale à laquelle le rayonnement ne dépend que de la température, correspondant à un rayonnement de surface, est de préférence entre 4.8 et 5 microns. Le procédé consiste à déterminer l'épaisseur du récipient entre les surfaces externe et interne en tant que fonction combinée desdites première et deuxième intensités mesurées. En d'autres termes, l'épaisseur et la température sont déterminées à partir des deux mesures de rayonnement prises dans la première bande spectrale et dans la deuxième bande spectrale.

Selon une variante de réalisation illustrée à la Fig. 3, ce brevet propose de répartir régulièrement sur la circonférence du récipient, quatre caméras mesurant le rayonnement dans la deuxième bande spectrale à laquelle le rayonnement ne dépend que de la température, et un pyromètre mesurant le rayonnement dans la première bande spectrale dont le signal dépend à la fois de la température de verre et de l'épaisseur.

Il apparaît donc que le calcul exact d'épaisseur n'est possible que pour un point mesuré par le pyromètre et la caméra. Il en résulte une relation établie pour connaître l'épaisseur en fonction de la température. Les autres points mesurés par les autres caméras ne sont qu'estimés par une extrapolation en faisant l'hypothèse que l'épaisseur dépend de la température selon un modèle mathématique local établi en un endroit du récipient. L'hypothèse que l'épaisseur se détermine par une mesure de température est fausse, sauf si les récipients sont homogènes en température lors du formage, autrement dit, sauf si les conditions initiales de température sont les mêmes pour toute la bouteille.

De manière complémentaire, il doit être noté que dans la première bande spectrale, le rayonnement du récipient par rapport au point de mesure du pyromètre, comprend le rayonnement de la paroi dite avant situé du côté du point de mesure et dépendant de son épaisseur et de sa température, mais également le rayonnement de la paroi opposée dite arrière qui émet vers l'intérieur du récipient et qui traverse la paroi avant. Ce « rayonnement arrière » se combine au « rayonnement avant » de la surface observée directement. La « paroi avant » n'absorbe que partiellement ce rayonnement dans la première bande spectrale. Ainsi, le rayonnement perçu dépend de l'épaisseur des deux parois et de la température des deux parois. Autrement dit, la mesure du rayonnement de la paroi avant ne permet pas de mesurer son épaisseur car le rayonnement est influencé par la paroi arrière.

Enfin, il s'avère que le procédé décrit par ce brevet est adapté pour mesurer l'épaisseur de verre d'un récipient relevant d'une famille de teintes limitées. Or il apparaît le besoin de pouvoir mesurer l'épaisseur de récipients en verre relevant du plus grand nombre de teintes possibles.

Exposé de l'invention

L'objet de l'invention vise donc à remédier aux inconvénients des techniques antérieures en proposant un nouveau procédé pour mesurer avec précision, l'épaisseur de la paroi de récipients en verre à haute température sortant de cavités de formage, en tenant compte de l'influence du rayonnement qu'une partie de la paroi apporte à une autre partie de la paroi.

Un autre objet de l'invention est de proposer un procédé pour mesurer avec précision, l'épaisseur de la paroi de récipients en verre présentant des teintes diversifiées.

Pour atteindre un tel objectif, le procédé pour mesurer l'épaisseur de récipients en verre à haute température sortant de cavités de formage, comprend les étapes suivantes :

- choisir de mesurer le rayonnement émis par le récipient depuis un premier côté et un deuxième côté du récipient diamétralement opposés de manière à prendre en compte le rayonnement émis par une première paroi du récipient située selon le premier côté et une deuxième paroi du récipient diamétralement opposé située selon le deuxième côté ;

- choisir de mesurer le rayonnement émis par le récipient dans une première bande spectrale dans une gamme comprise entre 2 800 nm et 4 000 nm et dans une deuxième bande spectrale, ces deux bandes spectrales étant distinctes et sélectionnées de manière que :

. d'une part l'absorption du rayonnement par le verre est différente dans les deux bandes spectrales pour la température des récipients ;
. et d'autre part au moins dans la première bande spectrale, l'absorption du rayonnement par le verre est telle que :

* le rayonnement mesuré depuis le premier côté du récipient, issu de la première paroi, est la somme du rayonnement émis par la première paroi et du rayonnement émis par la deuxième paroi transmis avec absorption au travers de la première paroi, de manière que ledit rayonnement combiné dépend des épaisseurs et des températures des premières et deuxièmes parois ;
* et le rayonnement mesuré depuis le deuxième côté du récipient, issu de la deuxième la paroi, est la somme du rayonnement émis par la deuxième paroi et du rayonnement émis par la première paroi et transmis avec absorption au travers de la deuxième paroi, de manière que ledit rayonnement combiné dépend des épaisseurs et des températures des premières et deuxièmes parois ;

- mesurer simultanément, depuis le premier côté du récipient, l'intensité du rayonnement issu de la première paroi dans la première bande spectrale et dans la deuxième bande spectrale et depuis le deuxième côté du récipient, l'intensité du rayonnement issu de la deuxième paroi dans la première bande spectrale et dans la deuxième bande spectrale ;
- et à déterminer au moins l'épaisseur de la première paroi et de la deuxième paroi, à partir des mesures d'intensité du rayonnement issu de la première paroi dans la première et la deuxième bandes spectrales et de la deuxième paroi dans la première et la deuxième bandes spectrales, en prenant en compte dans l'intensité du rayonnement dans la première bande spectrale, le rayonnement émis par une paroi et le rayonnement transmis avec absorption, et issu de l'autre paroi diamétralement opposée.

De plus, le procédé selon l'invention peut comporter en outre en combinaison au moins l'une et/ou l'autre des caractéristiques additionnelles suivantes :

- dans la deuxième bande spectrale, l'absorption du rayonnement par le verre est différente de celle de la première bande spectrale, et est telle que le rayonnement mesuré, d'une part, depuis le premier côté du récipient, issu de la première paroi est la somme du rayonnement émis par la première paroi et du rayonnement émis par la deuxième paroi et transmis au travers de la première paroi, et d'autre part, depuis le deuxième côté du récipient, issu de la deuxième paroi est la somme du rayonnement émis par la deuxième paroi et du rayonnement émis par la première paroi et transmis au travers de la deuxième paroi, le rayonnement combiné étant dépendant des épaisseurs des parois et des températures des parois ;
- on détermine également la température de la première paroi et de la deuxième paroi, à partir des mesures d'intensité du rayonnement de la première paroi dans la première et la deuxième bande spectrale et de la deuxième paroi dans la première et la deuxième bande spectrale, en prenant en compte dans l'intensité du rayonnement dans la première bande spectrale, le rayonnement transmis avec absorption, et issu de la paroi située de l'autre côté.

Un autre objet de l'invention est de proposer un procédé pour mesurer avec précision, l'épaisseur de la paroi de récipients en verre à haute température, pour une large gamme de teintes de verre y compris le verre blanc.
Pour atteindre un tel objectif, le procédé selon lequel on choisit de mesurer le rayonnement émis par le récipient dans la première bande spectrale dans une gamme comprise entre 3 000 nm et 4 000 nm.
De plus, le procédé selon l'invention peut comporter en outre en combinaison au moins l'une et/ou l'autre des caractéristiques additionnelles suivantes :

- dans la deuxième bande spectrale, l'absorption du rayonnement par le verre est telle que le rayonnement mesuré d'une part, depuis le premier côté du récipient, issu de la première paroi est le rayonnement émis seulement par la surface de la première paroi et d'autre part, depuis le deuxième côté du récipient, issu de la deuxième paroi est le rayonnement émis seulement par la surface de la deuxième paroi, le rayonnement dépendant seulement de la température ;

- on détermine les températures de la première paroi et de la deuxième paroi, à partir respectivement des mesures d'intensité du rayonnement de la première paroi dans la deuxième bande spectrale et de la deuxième paroi dans la deuxième bande spectrale ;
- on choisit de mesurer le rayonnement émis par le récipient dans la deuxième bande spectrale dans une gamme comprise entre 1 100 nm et 2 600 nm ;
- on choisit de mesurer le rayonnement émis par le récipient dans la deuxième bande spectrale dans une gamme supérieure à 4 500 nm et de préférence supérieure à 5 000 nm ;
- on mesure simultanément le rayonnement à l'aide d'au moins deux caméras infrarouge bi-spectrale délivrant chacune, pour chaque récipient, au moins deux images infrarouge du rayonnement de la paroi du récipient situé dans son champ d'observation.

Un autre objet de l'invention est de proposer une installation pour mesurer avec précision, l'épaisseur des parois en verre de récipients.

Pour atteindre un tel objectif, l'installation pour mesurer l'épaisseur de parois de récipients en verre à haute température sortant de cavités de formage et déplacés selon une trajectoire en translation, comporte :

- au moins une première et une deuxième caméras infrarouge bi-spectrale disposées diamétralement opposée de part et d'autre de la trajectoire des récipients pour prendre en compte le rayonnement émis par une première paroi du récipient situé d'un premier côté du récipient et une deuxième paroi du récipient situé d'un deuxième côté diamétralement opposé, chaque caméra délivrant deux images infrarouge du rayonnement de la paroi du récipient située dans son champ d'observation dans une première bande spectrale dans une gamme comprise entre 2 800 nm et 4 000 nm et dans une deuxième bande spectrale, ces deux bandes spectrales étant distinctes et sélectionnées de manière que :

     . d'une part l'absorption du rayonnement par le verre est différente dans les deux bandes spectrales pour la température des récipients ;
     . et d'autre part au moins dans la première bande spectrale, l'absorption du rayonnement par le verre est telle que :

          * le rayonnement mesuré depuis le premier côté du récipient, issu de la première paroi, est la somme du rayonnement émis par la première paroi et du rayonnement émis par la deuxième paroi et transmis avec absorption au travers de la première paroi, de manière que ledit rayonnement combiné dépend des épaisseurs et des températures des premières et deuxièmes parois ;
          * et le rayonnement mesuré depuis le deuxième côté du récipient, issu de la deuxième la paroi, est la somme du rayonnement émis par la deuxième paroi et du rayonnement émis par la première paroi et transmis avec absorption au travers de la deuxième paroi, de manière que ledit rayonnement combiné dépend des épaisseurs et des températures des première et deuxième parois ;

- un système pour piloter le fonctionnement des caméras infrarouges bi-spectrale de manière à acquérir simultanément, avec la première caméra, deux images mesurant l'intensité du rayonnement de la première paroi dans la première bande spectrale et dans la deuxième bande spectrale et avec la deuxième caméra, deux images mesurant l'intensité du rayonnement de la deuxième paroi dans la première bande spectrale et dans la deuxième bande spectrale ;
- et un calculateur configuré pour déterminer au moins les épaisseurs de la première paroi et de la deuxième paroi, en analysant les deux images donnant respectivement les mesures d'intensité du rayonnement issu de la première paroi dans la première et la deuxième bandes spectrales et les deux images de la deuxième paroi dans la première et la deuxième bande spectrale, en prenant en compte dans l'intensité du rayonnement dans la première bande spectrale, le rayonnement émis par une paroi et le rayonnement transmis avec absorption, et issu de la paroi située de l'autre côté.

De plus, l'installation selon l'invention peut comporter selon un mode de réalisation :

- une caméra infrarouge bi-spectrale qui comporte :
- un séparateur de faisceaux, en aval duquel les rayons sont séparés en deux faisceaux avals distincts ;
- en aval du séparateur de faisceaux, deux capteurs distincts ou deux portions de capteur, placés dans un plan ou deux plans image, recevant chacun(e) un des deux faisceaux avals distincts, le premier capteur ou la première portion de capteur recevant un premier faisceau de rayonnement dans la première bande spectrale et le deuxième capteur ou la deuxième portion de capteur recevant un deuxième faisceau de rayonnement dans la deuxième bande spectrale ;

- les premier et deuxième faisceaux étant mis en forme en amont ou en aval du séparateur, par un objectif formant par conjugaison optique sur chaque plan image, une image optique du récipient dans respectivement la première bande spectrale et la deuxième bande spectrale ;
- le premier et/ou le deuxième faisceau étant filtré(s) par un ou des filtres optiques sélectionnant respectivement la première bande spectrale et la deuxième bande spectrale.

Selon un autre mode de réalisation, chaque caméra infrarouge bi-spectrale comprend :

- un objectif formant par conjugaison optique sur un plan capteur, une image optique d'un champ traversé par un récipient ;
- deux portions linéaires de capteur distinctes, avec leurs lignes support verticales et disposées de manière que durant le défilement d'un récipient dans le champ d'un objectif, une image par balayage est réalisée avec chacune des deux portions linéaires de capteur ;
- la première portion linéaire de capteur recevant une première portion de faisceau de rayonnement dans la première bande spectrale ;
- la deuxième portion de capteur recevant une deuxième portion de faisceau de rayonnement dans la deuxième bande spectrale ;
- au moins un filtre optique disposé sur le trajet des faisceaux lumineux, pour sélectionner la première bande spectrale et la deuxième bande spectrale.

Brève description des dessins

[Fig. 1] La Figure 1 est une vue générale de dessus montrant une installation d'inspection conforme à l'invention adaptée pour mesurer l'épaisseur de la paroi en verre de récipients sortant d'une machine de formage.

[Fig. 2] La Figure 2 est une vue schématique explicitant le principe de rayonnement d'un récipient par rapport à des points de mesure situés de chaque côté du récipient, dans une première bande spectrale.

[Fig. 3] La Figure 3 est une vue illustrant le rayonnement thermique d'un corps noir.

[Fig. 4] La Figure 4 est une vue illustrant le rayonnement thermique d'un corps en verre.

[Fig. 5] La Figure 5 est une vue schématique explicitant le principe de rayonnement d'un récipient par rapport à des points de mesure situés de chaque côté du récipient, dans une deuxième bande spectrale.

[Fig. 6] La Figure 6 est une vue schématique explicitant le principe de rayonnement d'un récipient par rapport à des points de mesure situés de chaque côté du récipient, dans une deuxième bande spectrale sensible uniquement à la température de surface de la paroi en verre.

[Fig. 7] La Figure 7 est une vue schématique d'un exemple d'une caméra infrarouge bi-spectrale utilisée dans le cadre de l'invention.

[Fig. 8] La Figure 8 est une vue schématique d'un autre exemple d'une caméra infrarouge bi-spectrale utilisée dans le cadre de l'invention.

[Fig. 9] La Figure 9 est une vue schématique d'un autre exemple d'une caméra infrarouge bi-spectrale utilisée dans le cadre de l'invention.

Description des modes de réalisation

Dans la description de l'invention, sont utilisées une première bande spectrale $\lambda 1$ et une deuxième bande spectrale $\lambda 2$. Une bande spectrale $\lambda$ est un intervalle de longueur d'onde centré sur une valeur. Chaque bande spectrale de travail est choisi selon l'invention ou les variantes, dans des gammes de longueurs d'onde spécifiques, qui sont des intervalles de longueur d'onde plus larges. Cela signifie que l'intervalle de longueur d'onde de chaque bande spectrale de travail est inclus dans une gamme de longueurs d'ondes précise.

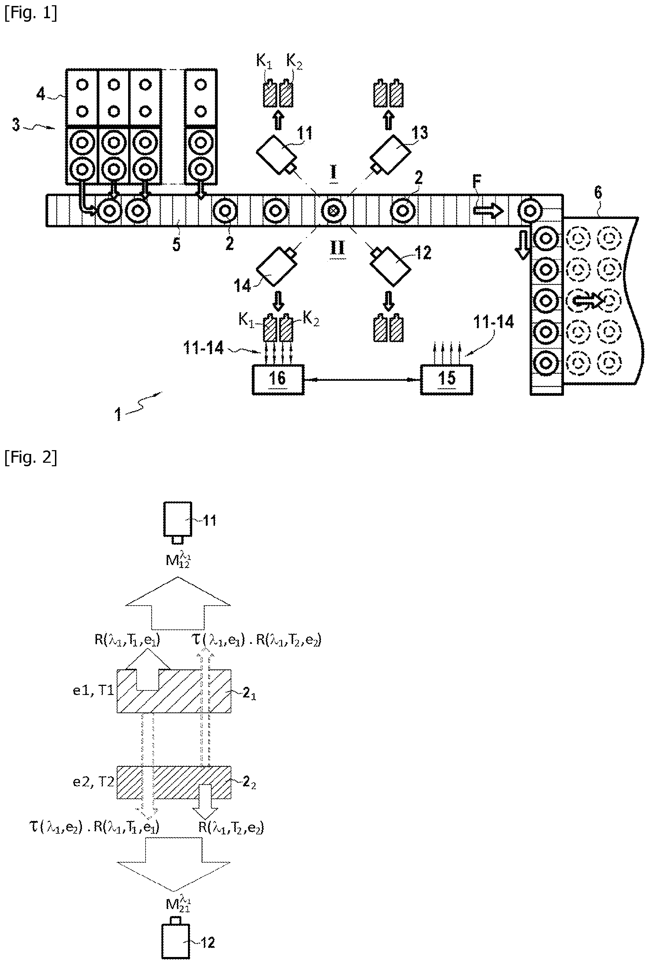

Tel que cela ressort plus précisément de la Fig. 1, l'objet de l'invention concerne une installation 1 permettant d'inspecter à chaud des récipients en verre 2 tels que par exemple des bouteilles ou des flacons, en vue de mesurer l'épaisseur de la paroi en verre de ces récipients. L'installation 1 est placée de manière à permettre d'inspecter les récipients 2 sortant d'une machine de fabrication ou de formage 3 de tous types connus en soi. En sortie de la machine de formage, les récipients 2 présentent une haute température typiquement comprise entre 300°C et 700°C.

La machine de formage 3 comporte de manière classique une série de cavités 4 assurant chacune le formage d'un récipient 2. De manière connue, les récipients 2 qui viennent d'être formés par la machine 3 sont posés successivement sur un convoyeur de sortie 5 pour former une file de récipients. Les récipients 2 sont transportés en file par le convoyeur 5 selon une trajectoire en translation F afin de les acheminer successivement à différents postes de traitement.

Conformément à une disposition avantageuse de l'invention mais non exclusive, l'installation 1 selon l'invention est placée au plus près de la machine de formage 3 de sorte que le convoyeur de sortie 5 assure le défilement successif des récipients 2 à haute température devant cette installation d'inspection 1. Typiquement, l'installation 1 est positionnée entre la sortie de la machine de formage 3 et l'arche de recuisson 6, et de préférence avant une hotte de traitement de surface constituant généralement le premier des postes de traitement après le formage.

L'installation 1 selon l'invention comporte au moins une première caméra infrarouge bi-spectrale 11 et une deuxième caméra infrarouge bi-spectrale 12 disposées diamétralement opposée de part et d'autre de la trajectoire de translation F des récipients. Chaque caméra infrarouge bi-spectrale 11, 12 est adaptée pour délivrer des images obtenues du rayonnement infrarouge de la paroi du récipient situé dans son champ d'observation. Chaque caméra infrarouge bi-spectrale 11, 12 délivre pour chaque récipient 2, au moins une première image obtenue du rayonnement infrarouge reçu dans une première bande spectrale $\lambda 1$ et au moins une deuxième image obtenue du rayonnement infrarouge reçu dans une deuxième bande spectrale $\lambda 2$. Une bande spectrale désigne un intervalle de longueurs d'ondes. Les caractéristiques des bandes spectrales $\lambda 1$, $\lambda 2$ seront précisées dans la suite de la description.

Selon la variante de réalisation illustrée sur la Fig. 1, l'installation 1 selon l'invention comporte également une deuxième paire 13, 14 de caméras infrarouge bi-spectrale. Ces caméras infrarouges bi-spectrale 13, 14 de cette deuxième paire sont également disposées diamétralement opposée de part et d'autre de la trajectoire de translation F des récipients. Par exemple, les caméras infrarouges bi-spectrales 11, 12 de la première paire sont disposées de manière que leurs axes d'observation soient à 45°par rapport à la trajectoire de translation F. De même, les caméras infrarouges bi-spectrales 13, 14 de la deuxième paire sont disposées à 45°par rapport à la trajectoire de translation F de sorte que les axes d'observations des caméras infrarouges bi-spectrale 11 à 14 sont décalés deux à deux de 90°. Bien entendu, une telle disposition des caméras infrarouges bi-spectrale n'est nullement limitative. Pour s'adapter à des conditions d'espacement défavorable entre les récipients en défilement, les angles entre les quatre axes d'observations peuvent être adaptés, par exemple aux valeurs suivantes : 30°, 150°, 30°,150°.

L'invention pourrait également fonctionner en utilisant trois caméras infrarouges bi-spectrales avec leurs axes à 120°. On considérera dans ce cas que le champ de chaque caméra, est partagé en deux portions, chaque portion de champ contenant l'image d'un secteur de 60° du récipient cylindrique. Chaque portion de champ d'une caméra est opposée à une portion de champ d'une autre caméra.

L'invention peut enfin être réalisée avec plus de quatre caméras, en respectant le principe de réaliser des images infrarouges bi-spectrales du point de vue de toutes les parois.

L'installation 1 selon l'invention comporte également un système 15 pour piloter le fonctionnement des caméras infrarouges bi-spectrales de manière à acquérir des images $K_1$, $K_2$ délivrées par les caméras infrarouges bi-spectrales selon un procédé qui sera décrit en détail dans la suite de la description. L'installation 1 selon l'invention comporte également un calculateur 16 configuré pour déterminer l'épaisseur de la paroi en verre du récipient en analysant les images $K_1$, $K_2$ délivrées par les caméras infrarouges bi-spectrale 11-14.

Dans le présent texte, un calculateur 16 est une unité informatique qui peut comprendre de manière connue notamment un microprocesseur, des bus d'entrée/sortie de données, de la mémoire, des connexions à un réseau informatique, et/ou un afficheur. Le calculateur peut être une unité informatique dédiée à l'installation pour mesurer l'épaisseur de parois, ou qui peut être partagée avec d'autres éléments de la ligne de fabrication de récipients. Il peut s'agir par exemple d'une unité centralisée de pilotage de la ligne ou d'une partie de celle-ci. Parmi les entrées-sorties, on inclut évidemment les moyens d'acquisition des images infrarouge. Parmi la mémoire, on inclut les moyens de mémoriser les images infrarouges numériques. Le microprocesseur est configuré pour exécuter des programmes organisés pour réaliser les algorithmes mettant en oeuvre le procédé selon l'invention.

L'analyse informatique d'images numériques produit un résultat d'inspection qui peut comprendre un résultat binaire (vrai/faux, présent/absent, conforme/non-conforme, ...) et/ou un résultat qualitatif, voire quantitatif, par exemple sous la forme d'une ou plusieurs mesures. Le résultat d'inspection peut donc inclure non seulement des valeurs minimales et maximales de l'épaisseur de verre, mais également la répartition et des écarts verticaux ou horizontaux, ainsi que l'emplacement des zones minces ou épaisses, significative pour corriger le procédé. En plus de la détermination d'une répartition ou cartographie d'épaisseur, donc de répartition de matière dans le récipient inspecté, le résultat d'inspection peut également comporter des cartographies de température des récipients, le repérage des régions de contraintes thermiques forte lorsque des variations localement fortes de températures sont observées. De plus cette analyse peut inclure la détection de défauts d'aspect ou de composition comme la présence d'inclusions, des bulles, des plis ou de craquelures en surface, ou de défauts géométriques ou dimensionnels tels que des cols penchés, des écarts de dimensions externes.

Parmi les sorties du calculateur, on peut prévoir des lignes de communication en direction de tout système de contrôle de la machine de fabrication, dans le but de corriger les dérives du procédé en fonction des mesures réalisées.

La description qui suit est réalisée en tenant compte uniquement de la première paire des caméras infrarouges bi-spectrale 11, 12 en considérant que cette description peut s'appliquer pour la deuxième paire des caméras infrarouges bi-spectrale. La première caméra infrarouge bi-spectrale 11 est disposée selon un premier côté I du récipient 2 tandis que la deuxième caméra infrarouge bi-spectrale 12 est disposée selon un deuxième côté II du récipient diamétralement opposé.

Etant donné que chaque récipient 2 comporte une paroi de verre présentant une forme de révolution ou cylindrique, le positionnement diamétralement opposé des caméras infrarouges bi-spectrale 11, 12 par rapport à un récipient conduit à considérer que pour chaque point de mesure, le récipient 2 présente une paroi dite avant et une paroi dite arrière, les parois avant et arrière pour une caméra infrarouge bi-spectrale correspondant aux parois arrière et avant pour l'autre caméra. Dans l'exemple illustré plus particulièrement à la Fig. 2, chaque récipient 2 comporte par convention, une première paroi $2_1$ située du premier côté I du récipient c'est-à-dire située la plus proche de la première caméra infrarouge bi-spectrale 11 et une deuxième paroi $2_2$, située du deuxième côté II diamétralement opposé c'est-à-dire située la plus proche de la deuxième caméra infrarouge bi-spectrale 12. Ainsi, chaque caméra infrarouge bi-spectrale 11, 12 prend en compte le rayonnement émis par la paroi avant du récipient et éventuellement, le rayonnement émis par la paroi arrière du récipient et ayant traversé la paroi avant.

Conformément à l'invention, chaque caméra infrarouge bi-spectrale 11, 12 délivre pour chaque récipient, au moins deux images infrarouge du rayonnement du récipient situé dans son champ d'observation, l'une $K_1$ dans une première bande spectrale λ1 et l'autre $K_2$, dans une deuxième bande spectrale λ2. Cette première bande spectrale λ1 et cette deuxième bande spectrale λ2 sont choisies en fonction du principe de mesure décrit ci-après.

Il doit tout d'abord être considéré que la première bande spectrale λ1 et la deuxième bande spectrale λ2 sont distinctes ou disjointes c'est-à-dire sans aucune valeur commune. Selon une autre caractéristique, l'absorption du rayonnement par le verre est différente dans les deux bandes spectrales pour la température des récipients 2.

Il est rappelé ci-dessous la théorie du rayonnement thermique. Dans la suite de la description, on assimile le rayonnement par souci de simplification, au rayonnement infrarouge perçu dans un angle solide pour un observateur observant un corps rayonnant, par exemple une caméra thermique observant un récipient.

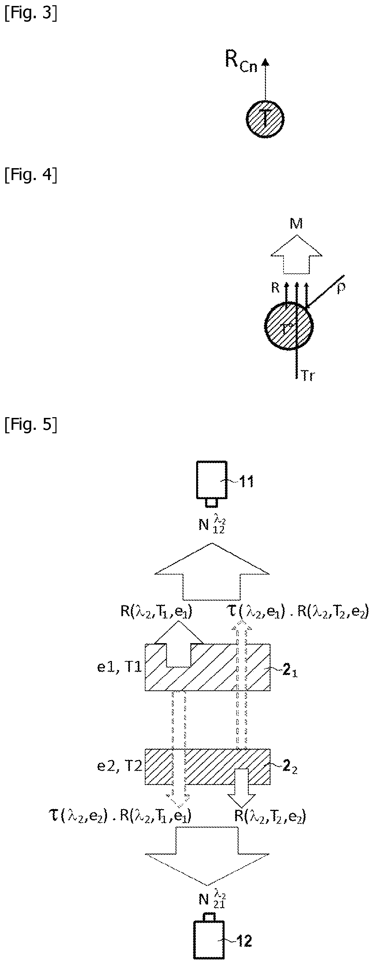

Comme illustré à la Fig. 3, le rayonnement thermique $R_{cn}$ du corps noir à une longueur d'onde donnée et une température donnée, λ et T respectivement, est donné par l'expression :

[Math. 1]

$$R_{cn}(\lambda, T) = \frac{C1}{\lambda^5} + \frac{1}{e^{C2/\lambda.T} - 1}$$

D'après la définition du corps noir, l'émissivité ε est, à l'équilibre thermique, égale à l'absorption α :

[Math. 2]

$$1 = \varepsilon = \alpha$$

Pour une paroi en verre (corps gris), l'expression du rayonnement total *M* perçu (radiation totale perçue) tel qu'illustré à la Fig.4 s'écrit :

[Math. 3]

$$M = R + \rho + Tr$$

- Où, R est le rayonnement thermique, ρ le rayonnement réfléchi et Tr le rayonnement transmis.

Dans le domaine spectral de travail, donc pour les intervalles de longueurs d'ondes perçus par les capteurs selon l'invention, le rayonnement réfléchi est considéré comme négligeable par rapport à l'intensité de rayonnement émis par un récipient. Par la suite, le flux perçu par réflexion est considéré comme nul, F=ρ=0.

[Math. 4]

$$M = R + Tr$$

Pour un corps semi-transparent, on considère la loi de Beer-Lambert définissant l'absorption α, d'un rayonnement A en fonction de l'épaisseur e traversée par le rayonnement Ao.

[Math. 5]

$$Tr = \tau \times Ao = (1 - \alpha) \times Ao$$

- où Ao est le rayonnement incident et τ la transmission.

[Math. 6]

$$\varepsilon(\lambda, T, e) = \alpha(\lambda, T, e) = \left(1 - e^{-\mu(\lambda, T)e}\right) = \left(1 - \tau(\lambda, T, e)\right)$$

- où $\mu(\lambda T)$ coefficient d'absorption (en mm$^{-1}$) pour une longueur d'onde λ, et une température T (en °K) donnée. En pratique, $\mu(\lambda T)$ est le coefficient d'absorption intégré sur une bande de longueurs d'onde étroite et centrée sur une longueur d'onde λ.

Dans la suite, les dépendances vis-à-vis de la température de l'émissivité et de l'absorption sont considérées négligeables, dans le domaine des conditions d'application, c'est-à-dire pour les températures de verre et intervalles de longueur d'onde choisis.

Le rayonnement R émis par la paroi de verre d'épaisseur e et de température T pour une longueur d'onde donnée λ s'écrit :

[Math. 7]

$$R(\lambda, T, e) = \varepsilon(\lambda, e) \times R_{cn}(\lambda, T)$$

- où l'émissivité de la paroi s'exprime selon l'équation [6] :

[Math. 8]

$$\varepsilon(\lambda, e) = 1 - e^{-\mu(\lambda) \cdot e}$$

L'application de la théorie du rayonnement thermique, selon l'invention conduit à prendre en compte pour un récipient les deux parois de verre. En effet, pour un point de mesure considéré du premier côté par exemple, de la première paroi du récipient, le rayonnement reçu du récipient comprend le rayonnement de la première paroi située du côté du point de mesure additionné du rayonnement de la deuxième paroi opposée qui émet vers l'intérieur du récipient et qui traverse la première paroi. Ainsi, comme illustré à la Fig. 2, le rayonnement perçu $M_{12}^{\lambda 1}$ de la première paroi $2_1$, d'épaisseur e1 et de température T1 du récipient dans une première bande spectrale $\lambda 1$ sensible à l'épaisseur et la température, inclut le rayonnement thermique $R(\lambda 1, T1, e1)$ de ladite paroi et le rayonnement transmis $\tau(\lambda 1, e1)$, $R(\lambda 1, T2, e2)$ qui est le rayonnement thermique $R(\lambda 1, T2, e2)$ de la deuxième paroi $2_2$, d'épaisseur e2 et température T2 au moins partiellement absorbé par la paroi $2_1$, donc avec une transmission $\tau(\lambda 1, e1)$. De manière similaire, le rayonnement perçu $M_{21}^{\lambda 1}$ de la deuxième paroi $2_2$, d'épaisseur e2 et température T2 du récipient dans la première bande spectrale $\lambda 1$ sensible à l'épaisseur et la température, inclut le rayonnement thermique $R(\lambda 1, T2, e2)$ de ladite paroi et le rayonnement transmis $\tau(\lambda 1, e2)$, $R(\lambda 1, T1, e1)$ de la première paroi $2_1$, d'épaisseur e1 et température T1 au moins partiellement absorbé par la paroi $2_2$, donc avec une transmission $\tau(\lambda 1, e2)$. Dans ce qui précède, les transmissions $\tau(\lambda 1, e1)$ et $\tau(\lambda 1, e2)$ pour la longueur d'onde $\lambda 1$, dépendent de l'épaisseur de la paroi traversée selon l'équation [6], avec l'influence de la température négligeable.

De manière analogue, comme illustré à la Fig. 5, dans la deuxième bande spectrale $\lambda 2$ sensible à l'épaisseur et la température, le rayonnement reçu $N_{12}^{\lambda 2}$ de la première paroi $2_1$, d'épaisseur e1 et température T1 du récipient inclus le rayonnement thermique émis par ladite paroi dans la longueur d'onde $\lambda 2$, soit $R(\lambda 2, T1, e1)$ et le rayonnement $\tau(\lambda 2, e1)$, $R(\lambda 2, T2, e2)$ de la deuxième paroi $2_2$, d'épaisseur e2 et température T2 au moins partiellement absorbé par la paroi $2_1$ donc avec une transmission $\tau(\lambda 2, e1)$. De manière similaire, le rayonnement reçu $N_{21}^{\lambda 2}$ de la deuxième paroi $2_2$, d'épaisseur e2 et température T2 du récipient dans la deuxième bande spectrale $\lambda 2$ sensible à l'épaisseur et la température inclut le rayonnement de ladite paroi $R(\lambda 2, T2, e2)$ et le rayonnement $\tau(\lambda 2, e2)$. $R(\lambda 2, T1, e1)$ de la première paroi $2_1$, d'épaisseur e1 et température T1, au moins partiellement absorbé par la paroi $2_2$ donc avec une transmission $\tau(\lambda 2, e2)$.

Compte tenu de la théorie du rayonnement thermique rappelée ci-dessus, il est possible d'écrire les équations suivantes :

$$M_{12}^{\lambda 1} = R(\lambda 1, T1, e1) + \tau(\lambda 1, e1).R(\lambda 1, T2, e2)$$
[9]

$$M_{21}^{\lambda 1} = R(\lambda 1, T2, e2) + \tau(\lambda 1, e2).R(\lambda 1, T1, e1)$$
[10]

$$N_{12}^{\lambda 2} = R(\lambda 2, T1, e1) + \tau(\lambda 2, e1).R(\lambda 2, T2, e2)$$
[11]

$$N_{21}^{\lambda 2} = R(\lambda 2, T2, e2) + \tau(\lambda 2, e2).R(\lambda 2, T1, e1)$$
[12]

Ces équations [9], [10], [11] et [12] concernent les rayonnements considérés de part et d'autre du récipient à savoir pour deux parois d'épaisseur e1, e2 à la température T1 et T2 respectivement et séparées par une lame d'air. Il est rappelé que l'émissivité doit être différente pour les longueurs d'ondes $\lambda 1$ et $\lambda 2$, sans quoi évidemment le système n'avait que deux équations au lieu de quatre.

L'invention repose sur le fait que les quatre équations [9] à [12], permettent de connaître les quatre inconnues, à savoir pour deux parois l'épaisseur e1, e2 et la température T1 et T2 respectivement, à partir des quatre mesures de

rayonnement $M_{12}^{\lambda 1}$, $M_{21}^{\lambda 1}$, $N_{12}^{\lambda 2}$ et $N_{21}^{\lambda 2}$ fournies par des moyens de mesure tels que des caméras bi spectrales infrarouges observant les récipients chauds. Les équations sont comme il vient d'être expliqué, à partir des lois de Planck pour le rayonnement et de Beer-Lambert pour la transmission. La connaissance a priori des récipients et du matériau, ou bien des méthodes d'indentification des paramètres ou d'étalonnage, conduisent à déterminer précisément ces équations pour une production donnée de récipients.

Pour faciliter la mise en en oeuvre de l'invention, une méthode consiste à simplifier les équations, ce qui permet une identification simplifiée des paramètres et des calculs plus rapides en temps réel lors de l'implémentation de la méthode au moyen d'un calculateur analysant des images infrarouges. Dans ce qui va suivre, il va donc être défini les fonctions simplifiées de l'émissivité, du rayonnement du corps noir et de la transmission avec absorption, autour des points de fonctionnement, donc pour des bandes spectrales choisies, pour la transmission spectrale du verre, pour la gamme de température des récipients en sortie de machines de formage, donc entre 300 et 700°C, pour la gamme des épaisseur à mesurer, par exemple entre 0,5 et 5 mm.

Conformément à l'équation [7], le rayonnement émis par une paroi, donc le rayonnement propre d'une paroi en verre est :

$$\text{[Math. 13]}$$
$$\mathrm{R}(\lambda 1, T1, e1) = \varepsilon\,(\lambda 1, e1)\,\times\,\mathrm{R}_{cn}(\lambda 1, T1).$$

Selon l'invention, et selon l'équation [8], l'émissivité ε dans la première bande spectrale λ1 est une fonction de l'épaisseur du corps semi-transparent. Cette émissivité est donc différente de 1. Pour des valeurs d'épaisseurs de paroi à mesurer (par exemple 0,5 à 5 mm), l'émissivité pour la première bande spectrale λ1 est approchée par une fonction affine de l'épaisseur dont les paramètres sont identifiables par mesure et étalonnage.

$$\text{[Math. 14]}$$
$$\varepsilon\,(e1) = (a \cdot e1 + b)$$

avec les coefficients a, b dépendant de la longueur d'onde λ1.

Le rayonnement du corps noir sera décrit par une fonction G(λ1,T), plus simple que l'équation [1]. Pour la longueur d'onde de travail λ1, plus précisément pour la bande spectrale de travail centrée sur λ1, G(λ1,T)= G1(T) est une fonction uniquement de la température. G1(T) est un modèle simplifié du rayonnement d'un corps noir pour la bande spectrale λ1, issu de la loi de Planck, par exemple c'est une fonction polynomiale, puissance ou exponentielle. Dans la pratique, cette fonction G1 prend en compte toute la chaîne d'acquisition, et notamment la sensibilité spectrale du capteur et la transmission des composants optiques interposés entre le récipient et le capteur. Les paramètres de la fonction G1, par exemple les coefficients du polynôme, l'exposant, le coefficient de l'exponentielle, etc. sont déterminés de toute manière appropriée, notamment de manière expérimentale dans une phase d'étalonnage du dispositif de mesure selon l'invention. Évidemment pour la deuxième longueur d'onde λ2 est déterminée de la même manière, la fonction G(λ2,T) = G2(T).

Un modèle simplifié pour le rayonnement R émis par une paroi de verre à une longueur d'onde donnée, ou pour une bande spectrale donnée autour d'une longueur d'onde λ1, s'écrit donc maintenant de la manière suivante :

$$\text{[Math. 15]}\ \mathrm{R}(\lambda 1, T1, e1) = \mathrm{R}(T1, e1) = (a \cdot e1 + b) \times \mathrm{G1}(T1)$$

Dans cette expression les paramètres ou constantes de G1(T) et les coefficients a et b peuvent être déterminés expérimentalement, ou a priori selon notamment la composition du verre. Évidemment, le rayonnement de la même paroi pour une longueur d'onde λ2, ainsi que le rayonnement de l'autre paroi d'épaisseur e2 et température T2 s'écrivent de la même manière, soit :

[Math. 15]

$$R(\lambda 1, T1, e1) = R(T1, e1) = (a \cdot e1 + b) \times G1(T1)$$

[Math. 16]

$$R(\lambda 1, T2, e2) = R(T2, e2) = (a \cdot e2 + b) \times G1(T2)$$

| [Math. 17]

$$R(\lambda 2, T1, e1) = R(T1, e1) = (c \cdot e1 + d) \times G2(T1)$$

| [Math. 18]

$$R(\lambda 2, T2, e2) = R(T2, e2) = (c \cdot e2 + d) \times G2(T2)$$

La transmission $\tau$ d'un rayonnement issu d'une paroi arrière par la paroi avant d'épaisseur e = e1 ou e2, est linéarisée dans l'équation [19] suivante, en utilisant notamment les équations [6] et [14] autour du point de fonctionnement déterminé par une température donnée, la première bande spectrale centrée autour de la première longueur d'onde $\lambda 1$ et pour la gamme d'épaisseurs à mesurer:

[Math. 19]

$$\tau(\lambda 1, e). = e^{-\mu(\lambda 1)e} = 1 - \alpha = 1 - \varepsilon(e) = 1 - (a \cdot e + b)$$

De même, pour la deuxième bande spectrale centrée autour de $\lambda 2$ l'atténuation du rayonnement issu de la paroi avant par la paroi arrière est :

[Math. 20]

$$\tau(\lambda 2, e). = e^{-\mu(\lambda 2)e} = 1 - \alpha = 1 - \varepsilon(e) = 1 - (c \cdot e + d)$$

Les coefficients a et b, c et d sont obtenus par étalonnage ou connus a priori par toute méthode appropriée. Ils dépendent, d'un point de fonctionnement, notamment des longueurs d'onde $\lambda 1$ et $\lambda 2$ choisies, et de la zone de température des récipients inspectés et de la gamme d'épaisseurs à mesurer.

Pour la longueur d'onde $\lambda 1$, la radiation $M_{12}^{\lambda 1}$ ( correspond au rayonnement total de la première paroi additionné du rayonnement de la deuxième paroi modulé par l'absorption de la première paroi. On peut donc l'exprimer comme suit :

[Math.21]

$$M_{12}^{\lambda 1} = (a \cdot e1 + b) \times G1(T1)$$

$$+(1 - (a \cdot e1 + b)) \times (a \cdot e2 + b) \times G1(T2)$$

De même, la radiation $M_{21}^{\lambda 1}$ correspond au rayonnement total de la deuxième paroi additionné du rayonnement de la première paroi modulé par l'absorption de la deuxième paroi. On peut donc l'exprimer comme suit :

[Math.22]

$$M_{21}^{\lambda 1} = (a \cdot e2 + b) \times G1(T2)$$

$$+(1 - (a \cdot e2 + b)) \times (a \cdot e1 + b) \times G1(T1)$$

De la même manière pour la longueur d'onde $\lambda 2$, la radiation $N_{12}$ correspond au rayonnement total de la première paroi additionné du rayonnement de la deuxième paroi modulée par l'absorption de la première paroi. On peut donc exprimer $N_{12}$ tel que :

[Math.23]

$$N_{12}^{\lambda 2} = (c \cdot e1 + d) \times G2(T1)$$

$$+(1 - (c \cdot e1 + d)) \times (c \cdot e2 + d) \times G2(T2)$$

De même, on peut donc exprimer $N_{21}^{\lambda 2}$ tel que :

[Math.24]

$$N_{21}^{\lambda 2} = (c \cdot e2 + d) \times G2(T2)$$

$$+(1 - (c \cdot e2 + d)) \times (c \cdot e1 + d) \times G2(T1)$$

Dans cette première variante de l'invention, si la deuxième bande spectrale $\lambda 2$ est choisie pour que l'émissivité bien que différente de celle pour la première bande spectrale $\lambda 1$, dépend de l'épaisseur, alors les équations [9], [10], [11] et [12] peuvent être remplacées respectivement par les équations [21], [22], [23] et [24].
De manière complémentaire, les considérations suivantes sont à prendre en compte en raison notamment des caractéristiques présentées par les récipients 2 sortant de la machine de formage.
Il est à noter ainsi que dans une gamme déterminée de longueur d'onde, l'émissivité du verre est très peu sensible à la température de la paroi qui varie entre 300 à 700°C. Pour cette gamme déterminée de longueur d'ondes, le coefficient d'absorption (donc l'émissivité) ne dépend pas de la température, ou bien cette dépendance est négligeable. Ainsi, seul le coefficient d'absorption spectrale $\mu$, relie l'absorption à l'épaisseur, et relie de la même manière l'émissivité à l'épaisseur.
Selon une caractéristique de l'invention, la première bande spectrale $\lambda 1$ est choisie dans cette gamme de longueur d'ondes pour laquelle l'émissivité du verre ne dépend pas de la température. Ceci permet d'appliquer les équations [7] et [8].

De plus, pour une bande spectrale déterminée dans une gamme supérieure à 4 500 nm et de préférence supérieure à 5 000 nm, l'émissivité du verre est très proche de 1 c'est-à-dire considérée comme égale à 1 par approximation. Pour cette bande spectrale, le rayonnement est assimilé à celui du corps noir. Selon une variante préférée de réalisation, la deuxième bande spectrale $\lambda 2$ est choisie dans cette gamme de longueurs d'ondes, pour laquelle le rayonnement ne dépend pas de l'épaisseur, pour laquelle la paroi observée ne transmet pas le rayonnement de la face opposée, puisque l'absorption est proche de 1 également, autrement dit le verre est opaque dans cette gamme de longueurs d'onde. On en déduit les expressions du rayonnement perçu dans cette bande spectrale, pour chaque face de la paroi, soit :

[Math. 25]

$$N_1 = R(\lambda 2, T1, e1) = G(T1)$$

[Math. 26]

$$N_2 = R(\lambda 2, T2, e2) = G(T2)$$

Dans cette variante, donc, les équations [9], [10], [11] et [12] peuvent être remplacées respectivement par les équations [21], [22], [25] et [26]. Cette variante utilisant une longueur d'onde dans laquelle le rayonnement ne dépend pas de l'épaisseur simplifie les calculs pour résoudre le système de 4 équations à 4 inconnues, puis que les inconnues T1 et T2, donc les températures des deux parois, découlent immédiatement des équations [21] et [22].

Conformément à l'invention, la première bande spectrale $\lambda 1$ est choisie de manière que dans la première bande spectrale, l'absorption du rayonnement par le verre est telle que :

* le rayonnement mesuré depuis le premier côté du récipient 2, issu de la première paroi $2_1$, est la somme du rayonnement émis par la première paroi $2_1$ et du rayonnement émis par la deuxième paroi $2_2$ et transmis avec absorption au travers de la première paroi $2_1$, de manière que ledit rayonnement combiné dépend des épaisseurs et des températures des première et deuxième parois,
* et le rayonnement mesuré depuis le deuxième côté du récipient 2, issu de la deuxième la paroi $2_2$, est la somme du rayonnement émis par la deuxième paroi $2_2$, et du rayonnement émis par la première paroi $2_1$ et transmis avec absorption au travers de la deuxième paroi $2_2$, de manière que ledit rayonnement combiné dépend des épaisseurs et des températures des première et deuxième parois.

Il doit être compris que l'invention propose de mesurer le rayonnement infrarouge avec une première bande spectrale $\lambda 1$ à laquelle l'intensité du rayonnement dépend de l'épaisseur de la paroi de verre et de la température de surface de la paroi. Selon l'invention, la première bande spectrale $\lambda 1$ est choisie pour que l'émissivité dépende de l'épaisseur de la paroi, donc soit éloignée de 1, mais aussi suffisamment forte pour qu'un rayonnement suffisant soit mesuré. Il est recherché par ailleurs que cette émissivité varie peu avec la teinte du verre. Ainsi, cette première bande spectrale $\lambda 1$ est choisie pour que l'émissivité varie par exemple entre (environ) 0.3 et 0.7 pour le verre blanc lorsque l'épaisseur varie entre 1 et 5 mm, pour des températures de verre autour de 450°C, plus largement entre 300 et 700°C. Il est à noter que l'émissivité pour la même épaisseur et les mêmes températures en verre vert ou ambre varie autour des mêmes valeurs.

Par ailleurs cette première bande spectrale $\lambda 1$ est choisie pour obtenir une transmission avec absorption par la face avant, du rayonnement issu de la face arrière. Ceci permet en effet que dans les équations [10] et [11], les transmissions respectivement $\tau(\lambda 1, e1) = e^{-\mu(\lambda 1)e1}$ et $\tau(\lambda 1, e2) e^{-\mu(\lambda 1)e2}$ ne valent pas 1, donc l'atténuation n'est pas nulle. Une atténuation nulle ne correspondrait pas à un récipient normal. Avec une atténuation nulle, on ne pourrait mesurer que la somme des épaisseurs e1 + e2 des deux parois sans différencier les deux. Inversement, si l'absorption est totale $e^{-\mu(\lambda 1)e1} = 0$ ou $e^{-\mu(\lambda 1)e2} = 0$, cela signifie que le verre est opaque pour la première bande spectrale. Dans ce cas on ne mesurerait dans cette première bande spectrale, que la température de surface de la paroi avant, indépendamment de l'épaisseur.

Il est à noter que le rayonnement émis par le récipient dans la première bande spectrale $\lambda 1$ peut être choisi dans une gamme comprise entre 1 100 nm et 2 600 nm. Toutefois, cette gamme est adaptée pour des récipients en verre vert ou en verre ambre mais pas pour des récipients en verre blanc car dans cette gamme, l'émissivité du verre blanc (transparent) est très faible.

Conformément à l'invention, la première bande spectrale $\lambda 1$ est choisie dans une gamme comprise entre 2 800 nm et 4 000 nm et de préférence entre 3 000 nm et 4 000 nm. Cette première bande spectrale préférée est choisie afin de fonctionner pour un grand nombre de teintes de verre, y compris le verre blanc.

Comme indiqué ci-dessus, la deuxième bande spectrale $\lambda2$ est choisie de manière que l'absorption du rayonnement par le verre est différente de celle de la première bande spectrale $\lambda1$. Il est rappelé que l'émissivité varie entre 0.3 et 0.7 pour la première bande spectrale $\lambda1$ choisie. Selon une première variante de réalisation illustrée à la Fig. 5, la deuxième bande spectrale $\lambda2$ est choisie également de manière que le rayonnement mesuré, d'une part, depuis le premier côté du récipient, issu de la première paroi $2_1$ est la somme du rayonnement émis par la première paroi $2_1$ et du rayonnement émis par la deuxième paroi $2_2$ et transmis au travers de la première paroi, et d'autre part, depuis le deuxième côté du récipient, issu de la deuxième paroi $2_2$ est la somme du rayonnement émis par la deuxième paroi $2_2$ et du rayonnement émis par la première paroi $2_1$ et transmis au travers de la deuxième paroi $2_2$, le rayonnement combiné étant dépendant des épaisseurs des parois et des températures des parois.

Selon cette première variante de réalisation, le rayonnement émis par le récipient dans la deuxième bande spectrale $\lambda2$ est choisie dans une gamme comprise entre 1 100 nm et 2 600 nm. Dans cette variante, la transmission est forte et l'émissivité risque d'être faible pour certains verres, typiquement inférieure à 0,1 pour des épaisseurs de verre de 1 à 5 mm à 450°C. Le signal infrarouge étant restreint, la précision de mesure de l'épaisseur peut être insuffisante pour ces teintes de verre.

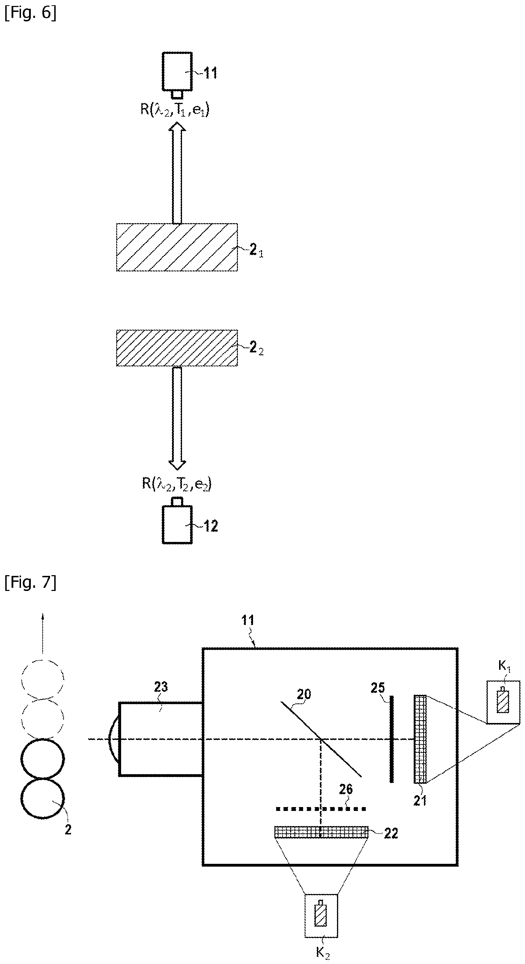

Selon une deuxième variante de réalisation préférée illustrée à la Fig. 6, la deuxième bande spectrale $\lambda2$ est choisie de manière que l'absorption du rayonnement par le verre est telle que le rayonnement mesuré d'une part, depuis le premier côté I du récipient, issu de la première paroi $2_1$ est le rayonnement émis seulement par la surface de la première paroi $2_1$ et d'autre part, depuis le deuxième côté II du récipient, issu de la deuxième paroi $2_2$ est le rayonnement émis seulement par la surface de la deuxième paroi, ledit rayonnement dépendant seulement de la température.

Ainsi, le rayonnement $N_1^{\lambda2}$ émis par la première paroi du récipient dans la deuxième bande spectrale est sensible à la température de surface uniquement. De même, le rayonnement $N_2^{\lambda2}$ émis par la deuxième paroi du récipient dans la deuxième bande spectrale est sensible à la température de surface uniquement.

Selon cette variante préférée de réalisation, la deuxième bande spectrale $\lambda2$ est choisie dans une gamme supérieure à 4 500 nm et de préférence supérieure à 5 000 nm. La deuxième bande spectrale $\lambda2$ est choisie pour que l'émissivité soit proche de 1 c'est-à-dire proche de celle du corps noir. Cela signifie que l'absorption du rayonnement par le verre dans cette bande spectrale est forte de sorte que l'émissivité est par exemple supérieure à 0,9. Le rayonnement N1, N2 perçu dans la deuxième bande spectrale $\lambda2$ pour la première face et pour la deuxième face est assez fidèlement représenté par la loi de Planck ou la fonction d'approximation selon l'invention G(T1) et G(T2), comme indiqué par les équations [25] et [26].

Selon cette variante préférée de réalisation avec la deuxième bande spectrale $\lambda2$ choisie dans une gamme supérieure à 4 500 nm et de préférence supérieure à 5 000 nm, il est avantageux de choisir simultanément la première bande spectrale $\lambda1$ dans une gamme comprise entre 2 800 nm et 4 000 nm et de préférence entre 3 000 nm et 4 000 nm. Ainsi, il devient possible pour mesurer ou réaliser des images dans les deux bandes spectrales $\lambda1$ et $\lambda2$, d'utiliser une seule et même technologie de capteur, de type capteur MWIR (Medium Wave InfraRed) de préférence non refroidi. Un capteur refroidi désigne ici les capteurs MWIR ou LWIR tels que ceux commercialisés par les sociétés SOFRADIR ou LYNRED qui sont équipés de systèmes refroidisseurs de type cryogénique. Un capteur non refroidi est en effet nettement moins cher qu'un capteur refroidi, il est plus robuste. Bien entendu les caméras MWIR selon l'invention sont équipées de solutions de protection des rayonnements (écran, lucarne, enceinte refroidis) et de refroidissement et/ou dissipation de chaleur tels que par exemple : circuits d'eau, ventilation forcée, cellules à effet Pelletier, caloducs, radiateurs etc...

Typiquement, la première bande spectrale $\lambda1$ est choisie dans une gamme comprise entre 3 000 nm et 4 000 nm et la deuxième bande spectrale $\lambda2$ est choisie dans une gamme supérieure à 4 500 nm. Avantageusement, la première bande spectrale $\lambda1$ est choisie centrée autour d'une valeur de longueur d'onde de l'ordre de 3600 nm alors que dans la deuxième bande spectrale $\lambda2$, est choisie centrée autour d'une valeur de longueur d'onde de l'ordre de 4700 nm.

Conformément à l'invention, les caméras infrarouges bi-spectrales 11-14 sont pilotées par le système 15 de manière à mesurer, depuis le premier côté I du récipient, l'intensité du rayonnement issu de la première paroi $2_1$ dans la première bande spectrale $\lambda1$ et simultanément dans la deuxième bande spectrale $\lambda2$ et depuis le deuxième côté II du récipient, l'intensité du rayonnement issu de la deuxième paroi $2_2$ dans la première bande spectrale $\lambda1$ et simultanément dans la deuxième bande spectrale $\lambda2$. Ainsi, pour chaque récipient 2, l'invention vise à réaliser au moins deux mesures de l'intensité du rayonnement reçu de deux parois opposées, dans une première bande spectrale et au moins deux mesures de l'intensité du rayonnement de deux parois opposées, dans une deuxième bande spectrale. Selon une caractéristique avantageuse, l'invention vise à réaliser comme mesure de rayonnement, des images monodimensionnelles ou bidimensionnelles des parois des récipients. Ainsi, les caméras infrarouges bi-spectrale 11, 14 délivrent chacune, pour chaque récipient, au moins deux images infrarouges du rayonnement de la paroi du récipient situé dans son champ d'observation. Selon une variante de réalisation, les deux caméras infrarouges bi-spectrales 11,12 délivrent pour chaque récipient, au moins deux images du rayonnement infrarouge dans la première bande spectrale et au moins deux images du rayonnement infrarouge dans la deuxième bande spectrale. Selon une autre variante de réalisation mettant en oeuvre quatre caméras infrarouges bi-spectrale 11, 14, il peut être obtenu huit images du rayonnement infrarouge afin que soit représenté dans les deux bandes spectrales, la paroi du récipient dans son ensemble. Dans ce cas, le champ observé est tel que chaque caméra mesure au moins un quart de la circonférence du récipient.

Il peut être envisagé également, d'augmenter le recouvrement des vues, au moyen de trois paires de caméras bi-spectrales opposées. Il peut être plus généralement prévu toute disposition des caméras en fonction de la forme et de l'espacement des récipients, afin d'observer complètement la circonférence des récipients, que leur forme soit caractérisée par une section plane horizontale circulaire (corps en forme de cône ou cylindre ordinaire) ou rectangulaire, polygonale etc. conformément aux pratiques de l'homme du métier et notamment selon la structure des systèmes d'inspection en ligne au secteur froid.

Conformément à une autre caractéristique de l'invention, le calculateur 16 permet de déterminer au moins l'épaisseur e1 de la première paroi $2_1$ et l'épaisseur e2 de la deuxième paroi $2_2$, à partir des mesures d'intensité du rayonnement issu de la première paroi $2_1$ dans la première et la deuxième bandes spectrales et de la deuxième paroi $2_2$ dans la première et la deuxième bandes spectrales, en prenant en compte dans l'intensité du rayonnement dans la première bande spectrale, le rayonnement émis par une paroi et le rayonnement transmis avec absorption, et issu de l'autre paroi diamétralement opposée.

Ainsi, l'épaisseur e1 de la première paroi $2_1$ et l'épaisseur e2 de la deuxième paroi $2_2$ sont déterminées et à partir des quatre mesures d'intensité du rayonnement, selon les équations générales [9], [10], [11] et [12], ou bien plus précisément leur simplification [21], [22], [23] et [24], ou bien encore [21], [22], [25] et [26] lorsque la deuxième longueur d'onde est choisie telle que l'émissivité est proche de 1. Ce système de quatre équations à quatre inconnues se résout au moins dans les versions linéarisées, mais également avec des modèles plus complexes si nécessaire dans le but d'augmenter la précision de mesure.

Selon la variante préférée de réalisation pour laquelle la deuxième bande spectrale $\lambda2$ dépend uniquement de la température, les épaisseurs et éventuellement les températures sont déterminées à partir du système d'équations, [21], [22], [25] et [26].

Il est rappelé que pour cette variante préférée de réalisation, le rayonnement infrarouge est mesuré d'une part avec une première bande spectrale à laquelle l'intensité du rayonnement dépend de l'épaisseur de la paroi et de la température de surface et d'autre part avec une deuxième bande spectrale à laquelle l'intensité du rayonnement dépend uniquement de la température de surface. Ainsi, l'information sur la température peut être « soustraite du signal ».

A partir des quatre mesures indépendantes du rayonnement et en prenant en compte dans la première bande spectrale, l'influence de la face opposée sur le rayonnement perçu pour chaque face, il est possible de déduire l'épaisseur e1 de la première face, l'épaisseur e2 de la deuxième face et éventuellement, la température T1 de la première face et la température T2 de la deuxième face. Pour réaliser ce calcul, on utilise un modèle mathématique reliant les quatre mesures de rayonnement et les quatre mesures finales à savoir les deux mesures d'épaisseur et les deux mesures de température.

Ce modèle mathématique peut être empirique ou analytique. Il peut être valable uniquement pour certaines conditions de fonctionnement permettant de fixer des constantes et de linéariser le modèle. Bien entendu, les simplifications opérées aux équations [15] et [19] ne sont pas essentielles à l'invention, elles permettent simplement une implémentation plus aisée et moins coûteuse des calculs. Le modèle analytique peut être évidemment plus élaboré, permettant des mesures plus précises, et prendre en compte la composition, les températures moyennes ou la forme des récipients. Le modèle mathématique peut comporter aussi un modèle géométrique apte à décrire la géométrie 3D d'un récipient, ayant comme caractéristiques une répartition d'épaisseur de verre et une répartition de température. Par exemple, pour un article simple de type conique, la surface du corps serait un cône, dont chaque point possède une valeur d'épaisseur en mm et une température en °K.

Il ressort de la description que l'installation 1 selon l'invention comporte des caméras infrarouges bi-spectrales 11-14. Bien que des capteurs d'images non plans peuvent être employés sans inconvénient pour réaliser l'invention à partir d'images non planes, la description suppose des capteurs plans et le terme d'image formée signifie d'une manière générale, une image plane d'un objet ou d'une scène, en l'occurrence d'au moins une partie de la paroi d'un récipient.

Lorsqu'il y a plusieurs capteurs par caméra, alors il existe plusieurs manières de les assembler. Dans ce qui suit l'amont et l'aval précisent la position des éléments optiques placés sur les faisceaux de rayonnement collectés et traités dans le sens de parcours de la lumière partant du récipient pour atteindre un capteur.

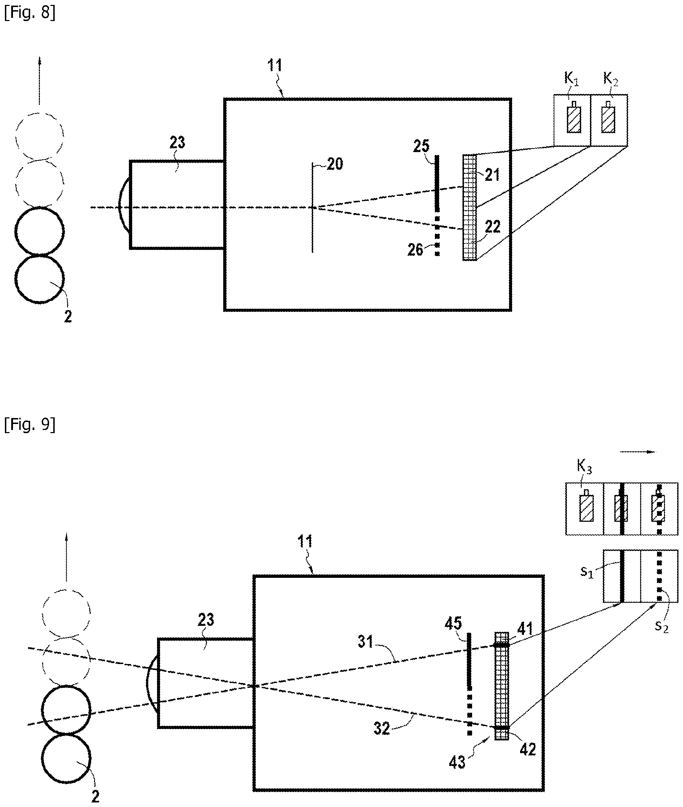

Selon une première variante de l'invention, une caméra infrarouge bi-spectrale comprend comme illustré par exemple aux Fig. 7, 8 :

- un séparateur de faisceaux 20, en aval duquel les rayons sont séparés en deux faisceaux avals distincts ;
- en aval du séparateur de faisceaux 20, deux capteurs 21, 22 distincts (Fig. 7) ou deux portions de capteur (Fig. 8), placés dans un plan ou deux plans image, recevant chacun(e) un des deux faisceaux avals distincts, le premier capteur ou la première portion de capteur recevant un premier faisceau de rayonnement dans la première bande spectrale et le deuxième capteur ou la deuxième portion de capteur recevant un deuxième faisceau de rayonnement dans la deuxième bande spectrale ;

- les premier et deuxième faisceaux étant mis en forme en amont ou en aval du séparateur 20, par un objectif 23 formant par conjugaison optique sur chaque plan image, donc chaque capteur ou portion de capteur, une image optique K1, K2 du récipient dans respectivement la première bande spectrale et la deuxième bande spectrale ;
- le premier et/ou le deuxième faisceau étant filtré(s) par un ou des filtres optiques, par exemple de type passe bande 25, 26 sélectionnant respectivement la première bande spectrale et la deuxième bande spectrale.

Les au moins deux capteurs 21, 22 distincts (Fig. 7) ou portions de capteur (Fig. 8), délivrent de chaque récipient chacun une image numérique correspondant à la conversion des images optiques K1, K2 du rayonnement M ou N perçu dans au moins deux bandes de longueur d'ondes infrarouges distinctes.

Le séparateur de faisceaux 20 est par exemple un prisme, une lame ou un cube séparateur. C'est un composant optique déviant un faisceau optique amont selon deux faisceaux optiques avals dans deux directions différentes.

Une portion linéaire de capteur est une ligne d'éléments photosensibles juxtaposés. L'acquisition ou la lecture d'une portion linéaire de capteur, fournit une seule ligne d'image numérique. Aussi, lors de l'inspection de récipients en mouvement, il est connu d'acquérir les lignes successives d'image numérique, afin de reproduire par la méthode simple et connue de balayage, une image bidimensionnelle d'un récipient traversant le champ plan d'une portion linéaire de capteur, *(nb champ plan = défini par ligne capteur et centre optique = champ de type* éventail*)*. Évidemment, le vecteur de déplacement ou défilement n'est pas parallèle à la direction de la portion linéaire de capteur. Il existe sur le marché des capteurs linéaires, comportant une seule ligne d'éléments photosensibles. Il existe aussi des capteurs comportant plusieurs lignes d'éléments photosensibles juxtaposées, et délivrant comme signal uniquement des lignes d'image numérique qui sont des combinaisons de l'information des différentes lignes. Enfin, on peut piloter un capteur matriciel de manière à n'acquérir qu'une ou deux ou plusieurs lignes d'image numériques issues de lignes distinctes du capteur, et à partir de ces lignes distinctes juxtaposées au cours du temps, obtenir par balayage une ou deux ou plusieurs images 2D du récipient en mouvement, séparées dans le temps, correspondant à des positions différentes. Autrement dit, un capteur matriciel peut être utilisé comme une ou deux ou plusieurs capteurs linéaires, il délivre dans le temps des lignes d'image numérique. La notion de portion linéaire de capteur recouvre les deux méthodes.

Selon une deuxième variante de l'invention chaque caméra infrarouge bi-spectrale comprend, comme illustré par exemple à la Fig. 9 :

- un objectif 23 formant par conjugaison optique sur un plan capteur, une image optique K3 d'un champ traversé par un récipient ;
- deux portions linéaires de capteur 41, 42 distinctes, avec leurs lignes support $s_1$, $s_2$ verticales et disposées de manière à ce que durant le défilement d'un récipient dans le champ d'un objectif 23, une image par balayage est réalisée avec chacune des deux portions linéaires de capteur ;
- la première portion linéaire de capteur 41 recevant une première portion de faisceau de rayonnement 31 dans la première bande spectrale ;

- la deuxième portion de capteur 42 recevant une deuxième portion de faisceau de rayonnement 32 dans la deuxième bande spectrale ;
- au moins un filtre optique 45 disposé sur le trajet des faisceaux lumineux, entre l'objectif et les deux portions linéaires de capteur 41, 42 pour sélectionner la première bande spectrale et la deuxième bande spectrale.

On note que le séparateur de faisceaux 20 n'est pas nécessaire dans cette version.

De cette manière, après qu'un récipient a traversé le champ de l'objectif 23, il a traversé le champ de chacune des deux portions linéaires de capteur. Par balayage on obtient deux images bidimensionnelles du récipient dans les deux longueurs d'ondes choisies.

Une façon avantageuse de réalisation est d'utiliser un seul capteur bidimensionnel 43, placé derrière au moins un filtre optique couvrant une seule partie du capteur, comme illustré à la Fig. 9. Une autre façon consiste à disposer deux capteurs linéaires dans le plan image.

Bien entendu dans cette deuxième variante, rien ne s'oppose à ce que, durant le défilement d'un récipient dans le champ de l'objectif 23, son image bidimensionnelle se trouve, à un moment donné, formée en partie sur la première et en partie sur la deuxième portion de capteur 41,42.

Dans toutes les variantes, éventuellement, deux filtres optiques sont utilisés pour sélectionner ces deux bandes spectrales. Il peut être avantageux de choisir comme filtres optique des filtres passe-bande.

Il est clair qu'au moins un filtre est nécessaire, seulement si les deux portions de capteurs sont de même technologie avec en conséquence chacune la même réponse ou sensibilité spectrale intrinsèque.

Selon une variante préférée dans laquelle la deuxième bande spectrale λ2 est choisie dans une gamme supérieure à 4 500 nm et de préférence supérieure à 5 000 nm, et la première bande spectrale λ1 dans une gamme comprise entre 2 800 nm et 4 000 nm et de préférence entre 3 000 nm et 4 000 nm, on peut utiliser pour le ou les capteurs un ou deux capteurs de type capteur MWIR (Medium Wave InfraRed) qui ne nécessitent aucun système de refroidissement comme expliqué ci-dessus. Ceci permet de réaliser les variantes illustrées en Fig. 8 et 9 comprenant un seul capteur. Pour réaliser des variantes comportant deux capteurs, comme illustré Fig. 7, l'utilisation de deux capteurs de même technologie simplifie la mise en oeuvre notamment en permettant que les deux capteurs aient la même résolution pour le même champ et soient synchrones, avec des moyens de pilotage communs et simplifiés.

Le ou les capteurs inclus dans chaque caméra infrarouge bi-spectrale sont par exemple à base de PbSe à 196 ou 300°K ou micro bolomètres.

Bien entendu, l'invention n'est pas limitée aux modes de réalisation de caméra bi spectrale décrits ci-dessus.

## Revendications

1. Procédé pour mesurer l'épaisseur de récipients (2) en verre à haute température sortant de cavités de formage, le procédé comprenant les étapes suivantes :

   - choisir de mesurer le rayonnement émis par le récipient (2) depuis un premier côté (I) et un deuxième côté (II) du récipient diamétralement opposés de manière à prendre en compte le rayonnement émis par une première paroi ($2_1$) du récipient située selon le premier côté et une deuxième paroi ($2_2$) du récipient diamétralement opposée située selon le deuxième côté ;
   - choisir de mesurer le rayonnement émis par le récipient (2) dans une première bande spectrale (λ1) dans une gamme comprise entre 2 800 nm et 4 000 nm et dans une deuxième bande spectrale (λ2), ces deux bandes spectrales étant distinctes et sélectionnées de manière que :

     . d'une part l'absorption du rayonnement par le verre est différente dans les deux bandes spectrales pour la température des récipients ;
     . et d'autre part au moins dans la première bande spectrale (λ1), l'absorption du rayonnement par le verre est telle que :

        * le rayonnement mesuré depuis le premier côté (I) du récipient, issu de la première paroi ($2_1$), est la somme du rayonnement émis par la première paroi ($2_1$) et du rayonnement émis par la deuxième paroi ($2_2$) transmis avec absorption au travers de la première paroi ($2_1$), de manière que ledit rayonnement combiné dépend des épaisseurs et des températures des premières et deuxièmes parois ($2_1$, $2_2$) ;
        * et le rayonnement mesuré depuis le deuxième côté (II) du récipient, issu de la deuxième la paroi ($2_2$), est la somme du rayonnement émis par la deuxième paroi ($2_2$) et du rayonnement émis par la première paroi ($2_1$) et transmis avec absorption au travers de la deuxième paroi ($2_2$), de manière que ledit rayonnement combiné dépend des épaisseurs et des températures des premières et deuxièmes parois ($2_1$, $2_2$) ;

   - mesurer simultanément, depuis le premier côté (I) du récipient, l'intensité du rayonnement issu de la première paroi ($2_1$) dans la première bande spectrale (λ1) et dans la deuxième bande spectrale (λ2) et depuis le deuxième côté (II) du récipient, l'intensité du rayonnement issu de la deuxième paroi ($2_2$) dans la première bande spectrale (λ1) et dans la deuxième bande spectrale (λ2) ;
   - et à déterminer au moins l'épaisseur de la première paroi et de la deuxième paroi ($2_2$), à partir des mesures d'intensité du rayonnement issu de la première paroi dans la première et la deuxième bandes spectrales et de la deuxième paroi dans la première et la deuxième bandes spectrales, en prenant en compte dans l'intensité du rayonnement dans la première bande spectrale, le rayonnement émis par une paroi et le rayonnement transmis avec absorption, et issu de l'autre paroi diamétralement opposée.

2. Procédé selon la revendication 1, selon lequel dans la deuxième bande spectrale (λ2), l'absorption du rayonnement par le verre est différente de celle de la première bande spectrale (λ1), et est telle que le rayonnement mesuré, d'une part, depuis le premier côté (I) du récipient, issu de la première paroi ($2_1$) est la somme du rayonnement émis par la première paroi ($2_1$) et du rayonnement émis par la deuxième paroi ($2_2$) et transmis au travers de la première paroi ($2_1$), et d'autre part, depuis le deuxième côté du récipient, issu de la deuxième paroi ($2_2$) est la somme du rayonnement émis par la deuxième paroi ($2_2$) et du rayonnement émis par la première paroi ($2_1$) et transmis au

travers de la deuxième paroi, le rayonnement combiné étant dépendant des épaisseurs des parois et des températures des parois.

3. Procédé selon l'une des revendications 1 ou 2, selon lequel on détermine également la température ($T_1$) de la première paroi ($2_1$) et de la deuxième paroi ($2_2$), à partir des mesures d'intensité du rayonnement de la première paroi ($2_1$) dans la première et la deuxième bande spectrale et de la deuxième paroi ($2_2$) dans la première et la deuxième bande spectrale, en prenant en compte dans l'intensité du rayonnement dans la première bande spectrale, le rayonnement transmis avec absorption, et issu de la paroi située de l'autre côté.

4. Procédé selon l'une des revendications 1 à 3, selon lequel on choisit de mesurer le rayonnement émis par le récipient dans la première bande spectrale ($\lambda 1$) dans une gamme comprise entre 3 000 nm et 4 000 nm.

5. Procédé selon l'une des revendications 1 et 4, selon lequel dans la deuxième bande spectrale ($\lambda 2$), l'absorption du rayonnement par le verre est telle que le rayonnement mesuré d'une part, depuis le premier côté (I) du récipient, issu de la première paroi ($2_1$) est le rayonnement émis seulement par la surface de la première paroi ($2_1$) et d'autre part, depuis le deuxième côté (II) du récipient, issu de la deuxième paroi ($2_2$) est le rayonnement émis seulement par la surface de la deuxième paroi ($2_2$), le rayonnement dépendant seulement de la température.

6. Procédé selon la revendication 5, dans lequel on détermine les températures ($T_1$, $T_2$) de la première paroi et de la deuxième paroi, à partir respectivement des mesures d'intensité du rayonnement de la première paroi ($2_1$) dans la deuxième bande spectrale ($\lambda 2$) et de la deuxième paroi ($2_2$) dans la deuxième bande spectrale ($\lambda 2$).

7. Procédé selon l'une des revendications 1 à 4, selon lequel on choisit de mesurer le rayonnement émis par le récipient dans la deuxième bande spectrale ($\lambda 2$) dans une gamme comprise entre 1 100 nm et 2 600 nm.

8. Procédé selon l'une des revendications 5 ou 6, selon lequel on choisit de mesurer le rayonnement émis par le récipient dans la deuxième bande spectrale ($\lambda 2$) dans une gamme supérieure à 4 500 nm et de préférence supérieure à 5 000 nm.

9. Procédé selon l'une des revendications précédentes, selon lequel on mesure simultanément le rayonnement à l'aide d'au moins deux caméras infrarouge bi-spectrales (11, 12 - 13, 14) délivrant chacune, pour chaque récipient, au moins deux images infrarouge du rayonnement de la paroi du récipient situé dans son champ d'observation.

10. Installation pour mesurer l'épaisseur de parois de récipients (2) en verre à haute température sortant de cavités de formage (4) et déplacés selon une trajectoire en translation (F), l'installation comportant :

- au moins une première (11) et une deuxième (12) caméras infrarouge bi-spectrales disposées diamétralement opposées de part et d'autre de la trajectoire (F) des récipients pour prendre en compte le rayonnement émis par une première paroi (21) du récipient (2) situé d'un premier côté (I) du récipient et une deuxième paroi ($2_2$) du récipient situé d'un deuxième côté diamétralement opposé, chaque caméra (11, 12) délivrant deux images du rayonnement infrarouge de la paroi du récipient située dans son champ d'observation dans une première bande spectrale ($\lambda 1$) dans une gamme comprise entre 2 800 nm et 4 000 nm et dans une deuxième bande spectrale ($\lambda 2$), ces deux bandes spectrales étant distinctes et sélectionnées de manière que :

. d'une part l'absorption du rayonnement par le verre est différente dans les deux bandes spectrales ($\lambda 1$, $\lambda 2$) pour la température des récipients ;
. et d'autre part au moins dans la première bande spectrale ($\lambda 1$), l'absorption du rayonnement par le verre est telle que :

* le rayonnement mesuré depuis le premier côté du récipient, issu de la première paroi ($2_1$), est la somme du rayonnement émis par la première paroi ($2_1$) et du rayonnement émis par la deuxième paroi ($2_2$) et transmis avec absorption au travers de la première paroi ($2_1$), de manière que ledit rayonnement combiné dépend des épaisseurs et des températures des premières et deuxièmes parois ;
* et le rayonnement mesuré depuis le deuxième côté (II) du récipient, issu de la deuxième la paroi ($2_2$), est la somme du rayonnement émis par la deuxième paroi ($2_2$) et du rayonnement émis par la première paroi ($2_1$) et transmis avec absorption au travers de la deuxième paroi ($2_2$), de manière que ledit rayonnement combiné dépend des épaisseurs et des températures des première et deuxième parois ($2_1$, $2_2$) ;

- un système (15) pour piloter le fonctionnement des caméras infrarouges bi-spectrale (11, 12) de manière à acquérir simultanément, avec la première caméra (11), deux images mesurant l'intensité du rayonnement de la première paroi ($2_1$) dans la première bande spectrale ($\lambda 1$) et dans la deuxième bande spectrale ($\lambda 2$) et avec la deuxième caméra (12), deux images mesurant l'intensité du rayonnement de la deuxième paroi ($2_2$) dans la première bande spectrale ($\lambda 1$) et dans la deuxième bande spectrale ($\lambda 2$) ;
- et un calculateur (16) configuré pour déterminer au moins les épaisseurs ($e_1$, $e_2$) de la première paroi ($2_1$) et de la deuxième paroi ($2_2$), en analysant les deux images donnant respectivement les mesures d'intensité du rayonnement issu de la première paroi ($2_1$) dans la première ($\lambda 1$) et la deuxième ($\lambda 2$) bandes spectrales et les deux images de la deuxième paroi dans la première et la deuxième bande spectrale, en prenant en compte dans l'intensité du rayonnement dans la première bande spectrale, le rayonnement émis par une paroi et le rayonnement transmis avec absorption, et issu de la paroi située de l'autre côté.

**11.** Installation selon la revendication 10, **caractérisée en ce qu'**une caméra infrarouge bi-spectrale (11- 14) comporte :

- un séparateur de faisceaux (20), en aval duquel les rayons sont séparés en deux faisceaux avals distincts ;
- en aval du séparateur de faisceaux (20), deux capteurs (21, 22) distincts ou deux portions de capteur, placés dans un plan ou deux plans image, recevant chacun(e) un des deux faisceaux avals distincts, le premier capteur ou la première portion de capteur recevant un premier faisceau de rayonnement dans la première bande spectrale et le deuxième capteur ou la deuxième portion de capteur recevant un deuxième faisceau de rayonnement dans la deuxième bande spectrale ;
- les premier et deuxième faisceaux étant mis en forme en amont ou en aval du séparateur (20), par un objectif (23) formant par conjugaison optique sur chaque plan image, une image optique (K1, K2) du récipient dans respectivement la première bande spectrale et la deuxième bande spectrale ;
- le premier et/ou le deuxième faisceau étant filtré(s) par un ou des filtres optiques (25, 26) sélectionnant respectivement la première bande spectrale et la deuxième bande spectrale.

**12.** Installation selon la revendication 10, **caractérisé en ce que** chaque caméra infrarouge bi-spectrale comprend :

- un objectif (23) formant par conjugaison optique sur un plan capteur, une image optique (K3) d'un champ traversé par un récipient ;
- deux portions linéaires de capteur (41, 42) distinctes, avec leurs lignes support ($s_1$, $s_2$) verticales et disposées de manière que durant le défilement d'un récipient dans le champ d'un objectif (23), une image par balayage est réalisée avec chacune des deux portions linéaires de capteur ;
- la première portion linéaire de capteur (41) recevant une première portion de faisceau de rayonnement (31) dans la première bande spectrale ;
- la deuxième portion de capteur (42) recevant une deuxième portion de faisceau de rayonnement (32) dans la deuxième bande spectrale ;
- au moins un filtre optique (45) disposé sur le trajet des faisceaux lumineux, pour sélectionner la première bande spectrale et la deuxième bande spectrale.

**13.** Installation selon l'une des revendications 11 ou 12, **caractérisée en ce que** l'un ou les filtres optiques (25, 26, 45) sélectionnent la première bande spectrale dans une gamme comprise entre 2 800 nm et 4 000 nm et la deuxième bande spectrale dans une gamme supérieure à 4 500 nm et de préférence supérieure à 5 000 nm.

**Patentansprüche**

**1.** Verfahren zur Messung der Dicke von Hochtemperatur-Glasbehältern (2), die aus Formnestern austreten, wobei das Verfahren die folgenden Schritte umfasst:

- Entscheiden, die Strahlung zu messen, die durch den Behälter (2) von einer ersten Seite (I) und einer zweiten Seite (II) des Behälters, die diametral gegenüberliegen, emittiert wird, sodass die Strahlung berücksichtigt wird, die durch eine erste Wand ($2_1$) des Behälters, die sich entlang der ersten Seite befindet, und eine zweite Wand ($2_2$) des Behälters, die diametral gegenüberliegt und sich entlang der zweiten Seite befindet, emittiert wird,
- Entscheiden, die Strahlung zu messen, die durch den Behälter (2) in einem ersten Spektralband ($\lambda 1$) in dem Bereich zwischen 2.800 nm und 4.000 nm und in einem zweiten Spektralband ($\lambda 2$) emittiert wird, wobei diese zwei Spektralbänder unterschiedlich und so ausgewählt sind, dass:

. einerseits die Absorption der Strahlung durch das Glas in den zwei Spektralbändern für die Temperatur der Behälter unterschiedlich ist,

. und andererseits zumindest in dem ersten Spektralband ($\lambda$1) die Absorption der Strahlung durch das Glas so ist, dass:

* die Strahlung, die von der ersten Seite (I) des Behälters gemessen und von der ersten Wand ($2_1$) ausgegeben wird, die Summe der Strahlung, die durch die erste Wand ($2_1$) emittiert wird, und der Strahlung, die durch die zweite Wand ($2_2$) emittiert und mit Absorption über die erste Wand ($2_1$) übertragen wird, ist, sodass die kombinierte Strahlung von den Dicken und den Temperaturen der ersten und der zweiten Wand ($2_1$, $2_2$) abhängt,
* und die Strahlung, die von der zweiten Seite (II) des Behälters gemessen und von der zweiten Wand ($2_2$) ausgegeben wird, die Summe der Strahlung, die durch die zweite Wand ($2_2$) emittiert wird, und der Strahlung, die durch die erste Wand ($2_1$) emittiert und mit Absorption über die zweite Wand ($2_2$) übertragen wird, ist, sodass die kombinierte Strahlung von den Dicken und den Temperaturen der ersten und der zweiten Wand ($2_1$, $2_2$) abhängt,

- gleichzeitiges Messen, von der ersten Seite (I) des Behälters, der Intensität der Strahlung, die von der ersten Wand ($2_1$) ausgegeben wird, in dem ersten Spektralband ($\lambda$1) und in dem zweiten Spektralband ($\lambda$2) und, von der zweiten Seite (II) des Behälters, der Intensität der Strahlung, die von der zweiten Wand ($2_2$) ausgegeben wird, in dem ersten Spektralband ($\lambda$1) und in dem zweiten Spektralband ($\lambda$2),

- und um zumindest die Dicke der ersten Wand und der zweiten Wand ($2_2$) aus den Messungen der Intensität der Strahlung zu bestimmen, die von der ersten Wand in dem ersten und dem zweiten Spektralband und von der zweiten Wand in dem ersten und dem zweiten Spektralband ausgegeben wird, indem bei der Intensität der Strahlung in dem ersten Spektralband die Strahlung, die durch eine Wand emittiert wird, und die Strahlung, die mit Absorption übertragen wird und von der anderen diametral gegenüberliegenden Wand ausgegeben wird, berücksichtigt werden.

2. Verfahren nach Anspruch 1, wobei sich in dem zweiten Spektralband ($\lambda$2) die Absorption der Strahlung durch das Glas von derjenigen des ersten Spektralbandes ($\lambda$1) unterscheidet und so ist, dass die Strahlung, die einerseits von der ersten Seite (I) des Behälters gemessen und von der ersten Wand ($2_1$) ausgegeben wird, die Summe der Strahlung, die durch die erste Wand ($2_1$) emittiert wird, und der Strahlung, die durch die zweite Wand ($2_2$) emittiert und über die erste Wand ($2_1$) übertragen wird, ist, und andererseits von der zweiten Seite des Behälters gemessen und von der zweiten Wand ($2_2$) ausgegeben wird, die Summe der Strahlung, die durch die zweite Wand ($2_2$) emittiert wird, und der Strahlung, die durch die erste Wand ($2_1$) emittiert und über die zweite Wand übertragen wird, ist, wobei die kombinierte Strahlung von den Dicken der Wände und den Temperaturen der Wände abhängt.

3. Verfahren nach einem der Ansprüche 1 oder 2, wobei auch die Temperatur ($T_1$) der ersten Wand ($2_1$) und der zweiten Wand ($2_2$) aus den Messungen der Intensität der Strahlung der ersten Wand ($2_1$) in dem ersten und dem zweiten Spektralband und der zweiten Wand ($2_2$) in dem ersten und dem zweiten Spektralband bestimmt wird, indem bei der Intensität der Strahlung in dem ersten Spektralband die Strahlung, die mit Absorption übertragen und von der Wand ausgegeben wird, die sich auf der anderen Seite befindet, berücksichtigt wird.

4. Verfahren nach einem der Ansprüche 1 bis 3, wobei entschieden wird, die Strahlung, die durch den Behälter in dem ersten Spektralband ($\lambda$1) emittiert wird, in einem Bereich zwischen 3.000 nm und 4.000 nm zu messen.

5. Verfahren nach einem der Ansprüche 1 und 4, wobei in dem zweiten Spektralband ($\lambda$2) die Absorption der Strahlung durch das Glas so ist, dass die Strahlung, die einerseits von der ersten Seite (I) des Behälters gemessen und von der ersten Wand ($2_1$) ausgegeben wird, die Strahlung ist, die nur durch die Oberfläche der ersten Wand ($2_1$) emittiert wird, und andererseits von der zweiten Seite (II) des Behälters gemessen und von der zweiten Wand ($2_2$) ausgegeben wird, die Strahlung ist, die nur durch die Oberfläche der zweiten Wand ($2_2$) emittiert wird, wobei die Strahlung nur von der Temperatur abhängt.

6. Verfahren nach Anspruch 5, wobei die Temperaturen ($T_1$, $T_2$) der ersten Wand und der zweiten Wand jeweils aus den Messungen der Intensität der Strahlung der ersten Wand ($2_1$) in dem zweiten Spektralband ($\lambda$2) und der zweiten Wand ($2_2$) in dem zweiten Spektralband ($\lambda$2) bestimmt werden.

7. Verfahren nach einem der Ansprüche 1 bis 4, wobei entschieden wird, die Strahlung, die durch den Behälter in dem zweiten Spektralband ($\lambda$2) emittiert wird, in einem Bereich zwischen 1.100 nm und 2.600 nm zu messen.

8. Verfahren nach einem der Ansprüche 5 oder 6, wobei entschieden wird, die Strahlung, die durch den Behälter in dem zweiten Spektralband ($\lambda$2) emittiert wird, in einem Bereich von mehr als 4.500 nm und vorzugsweise mehr als 5.000 nm zu messen.

9. Verfahren nach einem der vorhergehenden Ansprüche, wobei die Strahlung gleichzeitig mit Hilfe von zumindest zwei bispektralen Infrarotkameras (11, 12-13, 14) gemessen wird, die jeweils für jeden Behälter zumindest zwei Infrarotbilder der Strahlung von der Wand des Behälters, der sich in seinem Beobachtungsbereich befindet, liefern.

10. Anlage zur Messung der Dicke von Wänden von Hochtemperatur-Glasbehältern (2), die aus Formnestern (4) austreten und entlang einer Translationsbahn (F) bewegt werden, wobei die Anlage beinhaltet:

- zumindest eine erste (11) und eine zweite (12) bispektrale Infrarotkamera, die diametral gegenüberliegend auf beiden Seiten der Bahn (F) der Behälter angeordnet sind, um die Strahlung zu berücksichtigen, die durch eine erste Wand ($2_1$) des Behälters (2), die sich auf einer ersten Seite (I) des Behälters befindet, und eine zweite Wand ($2_2$) des Behälters, die sich auf einer diametral gegenüberliegenden zweiten Seite befindet, emittiert wird, wobei jede Kamera (11, 12) zwei Bilder der Infrarotstrahlung der Wand des Behälters, die sich in seinem Beobachtungsbereich befindet, in einem ersten Spektralband ($\lambda$1) in einem Bereich zwischen 2.800 nm und 4.000 nm und in einem zweiten Spektralband ($\lambda$2) liefert, wobei diese zwei Spektralbänder unterschiedlich und so ausgewählt sind, dass:

. einerseits die Absorption der Strahlung durch das Glas in den zwei Spektralbändern ($\lambda$1, $\lambda$2) für die Temperatur der Behälter unterschiedlich ist,
. und andererseits zumindest in dem ersten Spektralband ($\lambda$1) die Absorption der Strahlung durch das Glas so ist, dass:

* die Strahlung, die von der ersten Seite des Behälters gemessen wird und von der ersten Wand ($2_1$) ausgegeben wird, die Summe der Strahlung, die durch die erste Wand ($2_1$) emittiert wird, und der Strahlung, die durch die zweite Wand ($2_2$) emittiert und mit Absorption über die erste Wand ($2_1$) übertragen wird, ist, sodass die kombinierte Strahlung von den Dicken und den Temperaturen der ersten und der zweiten Wand abhängt,
* und die Strahlung, die von der zweiten Seite (II) des Behälters gemessen wird und von der zweiten Wand ($2_2$) ausgegeben wird, die Summe der Strahlung, die durch die zweite Wand ($2_2$) emittiert wird, und der Strahlung, die durch die erste Wand ($2_1$) emittiert und mit Absorption über die zweite Wand ($2_2$) übertragen wird, ist, sodass die kombinierte Strahlung von den Dicken und den Temperaturen der ersten und der zweiten Wand ($2_1$, $2_2$) abhängt,

- ein System (15) zum Steuern des Betriebs der bispektralen Infrarotkameras (11, 12), um gleichzeitig mit der ersten Kamera (11) zwei Bilder, welche die Intensität der Strahlung von der ersten Wand ($2_1$) in dem ersten Spektralband ($\lambda$1) und in dem zweiten Spektralband ($\lambda$2) messen, und mit der zweiten Kamera (12) zwei Bilder, welche die Intensität der Strahlung von der zweiten Wand ($2_2$) in dem ersten Spektralband ($\lambda$1) und in dem zweiten Spektralband ($\lambda$2) messen, zu erfassen,
- und einen Rechner (16), der dazu ausgestaltet ist, zumindest die Dicken ($e_1$, $e_2$) der ersten Wand ($2_1$) und der zweiten Wand ($2_2$) zu bestimmen, indem die zwei Bilder, die jeweils die Messungen der Intensität der Strahlung ergeben, die von der ersten Wand ($2_1$) in dem ersten ($\lambda$1) und dem zweiten ($\lambda$2) Spektralband ausgegeben wird, und die zwei Bilder der zweiten Wand in dem ersten und dem zweiten Spektralband analysiert werden, indem bei der Intensität der Strahlung in dem ersten Spektralband die Strahlung, die durch eine Wand emittiert wird, und die Strahlung, die mit Absorption übertragen und von der Wand, die sich auf der anderen Seite befindet, ausgegeben wird, berücksichtigt werden.

11. Anlage nach Anspruch 10, **dadurch gekennzeichnet, dass** eine bispektrale Infrarotkamera (11-14) beinhaltet:

- einen Strahlteiler (20), wobei stromabwärts davon die Strahlen in zwei unterschiedliche stromabwärts gerichtete Strahlbündel aufgeteilt werden,
- stromabwärts des Strahlteilers (20) zwei unterschiedliche Sensoren (21, 22) oder zwei Sensorteile, die in einer Bildebene oder zwei Bildebenen platziert sind und jeweils eines der zwei unterschiedlichen stromabwärts gerichteten Strahlbündel empfangen, wobei der erste Sensor oder der erste Sensorteil ein erstes Strahlbündel in dem ersten Spektralband empfängt und der zweite Sensor oder der zweite Sensorteil ein zweites Strahlbündel in dem zweiten Spektralband empfängt,

- wobei das erste und das zweite Strahlbündel stromaufwärts oder stromabwärts des Teilers (20) durch ein Objektiv (23) in Form gebracht werden, das durch optische Konjugation auf jeder Bildebene ein optisches Bild (K1, K2) des Behälters jeweils in dem ersten Spektralband und dem zweiten Spektralband formt,
- wobei das erste und/oder das zweite Strahlbündel durch einen oder mehrere optische Filter (25, 26) gefiltert wird/werden, die jeweils das erste Spektralband und das zweite Spektralband auswählen.

**12.** Anlage nach Anspruch 10, **dadurch gekennzeichnet, dass** jede bispektrale Infrarotkamera umfasst:

- ein Objektiv (23), das durch optische Konjugation auf einer Sensorebene ein optisches Bild (K3) eines Feldes formt, das von einem Behälter durchquert wird,
- zwei unterschiedliche lineare Sensorteile (41, 42), deren Stützlinien ($s_1$, $s_2$) vertikal und so angeordnet sind, dass während der Bewegung eines Behälters in dem Feld eines Objektivs (23) ein Bild pro Scan mit jedem der zwei linearen Sensorteile umgesetzt wird,
- wobei der erste lineare Sensorteil (41) einen ersten Teil des Strahlungsbündels (31) in dem ersten Spektralband empfängt,
- wobei der zweite Sensorteil (42) einen zweiten Teil des Strahlungsbündels (32) in dem zweiten Spektralband empfängt,
- zumindest einen optischen Filter (45), der auf der Bahn der Lichtbündel angeordnet ist, um das erste Spektralband und das zweite Spektralband auszuwählen.

**13.** Anlage nach einem der Ansprüche 11 oder 12, **dadurch gekennzeichnet, dass** einer oder die optischen Filter (25, 26, 45) das erste Spektralband in einem Bereich zwischen 2.800 nm und 4.000 nm und das zweite Spektralband in einem Bereich von mehr als 4.500 nm und vorzugsweise mehr als 5.000 nm auswählen.

**Claims**

**1.** A method for measuring the thickness of high-temperature glass containers (2) exiting the forming cavities, the method comprising the following steps:

- choosing to measure the radiation emitted by the container (2) from a first side (I) and a second side (II) of the container diametrically opposite to each other so as to take into account the radiation emitted by a first wall ($2_1$) of the container located along the first side and a diametrically opposite second wall ($2_2$) of the container located along the second side;
- choosing to measure the radiation emitted by the container (2) in a first spectral band ($\lambda 1$) in a range between 2,800 nm and 4,000 nm and in a second spectral band ($\lambda 2$), these two spectral bands being distinct and selected such that:

· on the one hand, the absorption of the radiation by the glass is different in the two spectral bands for the temperature of the containers;
· and on the other hand at least in the first spectral band ($\lambda 1$), the absorption of the radiation by the glass is such that:

\* the radiation measured from the first side (I) of the container, coming from the first wall ($2_1$), is the sum of the radiation emitted by the first wall ($2_1$) and of the radiation emitted by the second wall ($2_2$) transmitted with absorption through the first wall ($2_1$), such that said combined radiation depends on the thicknesses and temperatures of the first and second walls ($2_1$, $2_2$);
\* and the radiation measured from the second side (II) of the container, coming from the second wall ($2_2$), is the sum of the radiation emitted by the second wall ($2_2$) and of the radiation emitted by the first wall ($2_1$) and transmitted with absorption through the second wall ($2_2$), such that said combined radiation depends on the thicknesses and temperatures of the first and second walls ($2_1$, $2_2$);

- simultaneously measuring, from the first side (I) of the container, the intensity of the radiation coming from the first wall ($2_1$) in the first spectral band ($\lambda 1$) and in the second spectral band ($\lambda 2$) and from the second side ( II) of the container, the intensity of the radiation coming from the second wall ($2_2$) in the first spectral band ($\lambda 1$) and in the second spectral band ($\lambda 2$);
- and determining at least the thickness of the first wall and of the second wall ($2_2$), from the measurements of the intensity of the radiation coming from the first wall in the first and second spectral bands and from the second wall in the first and second spectral bands, by taking into account in the intensity of the radiation in the first spectral band, the radiation emitted by one wall and the radiation transmitted with absorption, and coming from the other diametrically opposite wall.

2. The method according to claim 1, according to which in the second spectral band ($\lambda$2), the absorption of the radiation by the glass is different from that of the first spectral band ($\lambda$1), and is such that the radiation measured, on the one hand, from the first side (I) of the container, coming from the first wall ($2_1$) is the sum of the radiation emitted by the first wall ($2_1$) and of the radiation emitted by the second wall ($2_2$) and transmitted through the first wall ($2_1$), and on the other hand, from the second side of the container, coming from the second wall ($2_2$) is the sum of the radiation emitted by the second wall ($2_2$) and of the radiation emitted by the first wall ($2_1$) and transmitted through the second wall, the combined radiation being dependent on the thicknesses of the walls and temperatures of the walls.

3. The method according to any of claims 1 or 2, according to which the temperature ($T_1$) of the first wall ($2_1$) and of the second wall ($2_2$) is also determined from the measurements of the intensity of the radiation of the first wall ($2_1$) in the first and the second spectral band and of the second wall ($2_2$) in the first and the second spectral band, by taking into account in the intensity of the radiation in the first spectral band, the radiation transmitted with absorption, and coming from the wall located on the other side.

4. The method according to any of claims 1 to 3, according to which it is chosen to measure the radiation emitted by the container in the first spectral band ($\lambda$1) in a range between 3,000 nm and 4,000 nm.

5. The method according to any of claims 1 and 4, according to which in the second spectral band ($\lambda$2), the absorption of the radiation by the glass is such that the radiation measured, on the one hand from the first side (I) of the container, coming from the first wall ($2_1$), is the radiation emitted only by the surface of the first wall ($2_1$), and on the other hand from the second side (II) of the container, coming from the second wall ($2_2$), is the radiation emitted only by the surface of the second wall ($2_2$), the radiation depending only on the temperature.

6. The method according to claim 5, wherein the temperatures ($T_1$, $T_2$ of the first wall and of the second wall are determined from respectively the measurements of the intensity of the radiation of the first wall ($2_1$) in the second spectral band ($\lambda$2) and of the second wall ($2_2$) in the second spectral band ($\lambda$2).

7. The method according to any of claims 1 to 4, according to which it is chosen to measure the radiation emitted by the container in the second spectral band ($\lambda$2) in a range between 1,100 nm and 2,600 nm.

8. The method according to any of claims 5 or 6, according to which it is chosen to measure the radiation emitted by the container in the second spectral band ($\lambda$2) in a range greater than 4,500 nm and preferably greater than 5,000 nm.

9. The method according to any of the preceding claims, according to which the radiation is simultaneously measured using at least two bispectral infrared cameras (11, 12 - 13, 14) each delivering, for each container, at least two infrared images of the radiation of the wall of the container located in its field of observation.

10. A facility for measuring the thickness of the walls of the high-temperature glass containers (2) exiting the forming cavities (4) and moved along a translational path (F), the facility including:

- at least a first (11) and a second (12) bispectral infrared camera disposed diametrically opposite to each other on either side of the path (F) of the containers to take into account the radiation emitted by a first wall ($2_1$) of the container (2) located on a first side (I) of the container and a second wall ($2_2$) of the container located on a diametrically opposite second side, each camera (11, 12) delivering two images of the infrared radiation of the wall of the container located in its field of observation in a first spectral band ($\lambda$1) in a range between 2,800 nm and 4,000 nm and in a second spectral band ($\lambda$2), these two spectral bands being distinct and selected such that:

· on the one hand, the absorption of the radiation by the glass is different in the two spectral bands ($\lambda$1, $\lambda$2) for the temperature of the containers;
· and on the other hand at least in the first spectral band ($\lambda$1), the absorption of the radiation by the glass is such that:

* the radiation measured from the first side of the container, coming from the first wall ($2_1$), is the sum of the radiation emitted by the first wall ($2_1$) and of the radiation emitted by the second wall ($2_2$) and

transmitted with absorption through of the first wall ($2_1$), such that said combined radiation depends on the thicknesses and temperatures of the first and second walls;

* and the radiation measured from the second side (II) of the container, coming from the second wall ($2_2$), is the sum of the radiation emitted by the second wall ($2_2$) and of the radiation emitted by the first wall ($2_1$) and transmitted with absorption through the second wall ($2_2$), such that said combined radiation depends on the thicknesses and temperatures of the first and second walls ($2_1$, $2_2$) ;

- a system (15) for driving the operation of the bispectral infrared cameras (11,12) so as to acquire simultaneously, with the first camera (11), two images measuring the intensity of the radiation of the first wall ($2_1$) in the first spectral band ($\lambda1$) and in the second spectral band ($\lambda2$) and with the second camera (12), two images measuring the intensity of the radiation of the second wall ($2_2$) in the first spectral band ($\lambda1$) and in the second spectral band ($\lambda2$);
- and a computer (16) configured to determine at least the thicknesses ($e_1$, $e_2$) of the first wall ($2_1$) and of the second wall ($2_2$), by analyzing the two images giving respectively the measurements of the intensity of the radiation coming from of the first wall ($2_1$) in the first ($\lambda1$) and the second ($\lambda2$) spectral band and the two images of the second wall in the first and the second spectral band, by taking into account in the intensity of the radiation in the first spectral band, the radiation emitted by a wall and the radiation transmitted with absorption, and coming from the wall located on the other side.

**11.** The facility according to claim 10, **characterized in that** a bispectral infrared camera (11-14) includes:

- a beam splitter (20), downstream of which the rays are separated into two distinct downstream beams;
- downstream of the beam splitter (20), two distinct sensors (21, 22) or two sensor portions, placed in a plane or two image planes, each receiving one of the two distinct downstream beams, the first sensor or the first sensor portion receiving a first radiation beam in the first spectral band and the second sensor or the second sensor portion receiving a second radiation beam in the second spectral band;
- the first and second beams being shaped upstream or downstream of the splitter (20), by a lens (23) forming by optical conjugation on each image plane, an optical image (K1, K2) of the container in respectively the first spectral band and the second spectral band;
- the first and/or the second beam being filtered by one or several optical filters (25, 26) selecting respectively the first spectral band and the second spectral band.

**12.** The facility according to claim 10, **characterized in that** each bispectral infrared camera comprises:

- a lens (23) forming, by optical conjugation on a sensor plane, an optical image (K3) of a field through which a container passes;
- two distinct linear sensor portions (41, 42), with their support lines ($s_1$, $s_2$) being vertical and disposed such that during the travel of a container in the field of a lens (23), a scan image is produced with each of the two linear sensor portions;
- the first linear sensor portion (41) receiving a first radiation beam portion (31) in the first spectral band;
- the second sensor portion (42) receiving a second radiation beam portion (32) in the second spectral band;
- at least one optical filter (45) disposed on the path of the light beams, to select the first spectral band and the second spectral band.

**13.** The facility according to any of claims 11 or 12, **characterized in that** the optical filter(s) (25, 26, 45) select the first spectral band in a range between 2,800 nm and 4,000 nm and the second spectral band in a range greater than 4,500 nm and preferably greater than 5,000 nm.

[Fig. 1]

[Fig. 2]

[Fig. 3]

$R_{Cn}$

$T$

[Fig. 4]

M

R    $\rho$

T

Tr

[Fig. 5]

11

$N_{12}^{\lambda_2}$

$R(\lambda_2, T_1, e_1)$    $\tau(\lambda_2, e_1) \cdot R(\lambda_2, T_2, e_2)$

e1, T1    $2_1$

e2, T2    $2_2$

$\tau(\lambda_2, e_2) \cdot R(\lambda_2, T_1, e_1)$    $R(\lambda_2, T_2, e_2)$

$N_{21}^{\lambda_2}$

12

[Fig. 6]

$R(\lambda_2,T_1,e_1)$

$2_1$

$2_2$

$R(\lambda_2,T_2,e_2)$

[Fig. 7]

[Fig. 8]

[Fig. 9]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3535522 A
- EP 0643297 A

- EP 1020703 A